# EUROPEAN PATENT SPECIFICATION

(11) **EP 3 377 476 B1**
(45) Date of publication and mention of the grant of the patent: **26.10.2022**
(21) Application number: 16866934.9
(22) Date of filing: 15.11.2016
(51) Int. Cl.: C07D 271/06, A61K 31/4245, C07B 59/00, A61P 25/00, A61P 11/00, A61P 37/04, A61P 3/00, A61P 21/00, A61P 9/00, A61P 25/28, A61P 29/00, A61P 35/00, A61P 17/00, A61P 19/02, A61P 27/02, A61P 7/00, A61P 25/10, A61P 3/10

(54) **HYDROGEN ISOTOPE-ENRICHED ANALOGUES OF 1,2,4-OXADIAZOLE BENZOIC ACID COMPOUNDS, COMPOSITIONS AND USES THEREOF**
MIT WASSERSTOFFISOTOPEN ANGEREICHERTE ANALOGA VON 1,2,4-OXADIAZOLBENZOESAÜREVERBINDUNGEN, ZUSAMMENSETZUNGEN UND VERWENDUNGEN DAVON
ANALOGUES ENRICHI EN ISOTOPES D'HYDROGÈNE DE COMPOSÉS D'ACIDE BENZOÏQUE 1,2,4-OXADIAZOLE, COMPOSITIONS ET LEURS UTILISATIONS

(30) Priority: 16.11.2015 US 201562255751 P
(43) Date of publication of application: 26.09.2018
(73) Proprietor: PTC Therapeutics, Inc., South Plainfield, New Jersey 07080 (US)
(72) Inventor: HWANG, Seongwoo, Edison, NJ 08817 (US); WELCH, Ellen, Califon, NJ 07830 (US)
(74) Representative: Jones Day
(86) International application number: PCT/US2016/061985
(87) International publication number: WO 2017/087364

(56) References cited:
- WO-A1-2015/063102
- WO-A1-2015/134711
- WO-A1-2016/073545
- US-A1- 2004 204 461
- US-A1- 2004 204 461
- US-A1- 2006 058 349
- US-A1- 2006 182 717
- US-A1- 2007 082 929
- US-A1- 2014 303 203
- US-A1- 2015 119 380
- FOSTER A B: "Deuterium isotope effects in the metabolism of drugs and xenobiotics: implications for drug design", ADVANCES IN DRUG RESEA, ACADEMIC PRESS, LONDON, GB, vol. 14, 1 January 1985 (1985-01-01), pages 1-40, XP009086953, ISSN: 0065-2490
- Jon M. Fukuto ET AL: "Determination of the mechanism of demethylenation of (methylenedioxy)phenyl compounds by cytochrome P450 using deuterium isotope effects", Journal of Medicinal Chemistry, vol. 34, no. 9, 1 September 1991 (1991-09-01), pages 2871-2876, XP055009050, ISSN: 0022-2623, DOI: 10.1021/jm00113a028
- FISHER MICHAEL B ET AL: "The complexities inherent in attempts to decrease drug clearance by blocking sites of CYP-mediated metabolism", CURRENT OPINION IN DRUG DISCOVERY AND DEVELOPMENT, CURRENT DRUGS, LONDON, GB, vol. 9, no. 1, 1 January 2006 (2006-01-01) , pages 101-109, XP009130718, ISSN: 1367-6733

## Description

### 1. FIELD OF THE INVENTION

Provided herein are hydrogen isotope-enriched analogues of 1,2,4-oxadiazole benzoic acid compounds and compositions thereof comprising the compounds and the compounds for use in methods for treating or preventing a nonsense mutation mediated disease.

### 2. BACKGROUND OF THE INVENTION

Gene expression in cells depends upon the sequential processes of transcription and translation. Together, these processes produce a protein from the nucleotide sequence of its corresponding gene.

Insertions, deletions, transition and transversion mutations of a DNA sequence can all result in a nonsense mutation, or chain termination mutation, in which the base mutation or frameshift mutation changes an amino acid codon into one of the three stop codons. The resulting premature stop codons in the mRNA transcript can produce aberrant proteins in cells as a result of premature translation termination. A nonsense mutation in an essential gene can be lethal and can also result in a number of diseases, such as, cancers, lysosomal storage disorders, the muscular dystrophies, cystic fibrosis and hemophilia, to name a few.

Small molecule therapeutics that suppress premature translation termination by mediating ribosomal "readthrough" of the premature stop codon would be useful for the treatment of a number of diseases. The discovery of small molecule drugs, particularly orally bioavailable drugs, may lead to the introduction of selective therapeutics which can be used against a broad spectrum of diseases caused by nonsense mutations.

Isotopic enrichment (*e.g*., deuteration, triteration, etc.) of pharmaceuticals to improve pharmacokinetics ("PK"), pharmacodynamics ("PD"), and toxicity profiles, has been demonstrated previously with some classes of drugs. (*See, e.g.,* Lijinsky et al., Food Cosmet. Toxicol., Vol. 20, No. 4, p. 393 (1982); Lijinsky et al., J. Nat. Cancer Inst., Vol. 69, No. 5, p. 1127 (1982); Mangold et al., Mutation Res. Vol. 308, No. 1, p. 33 (1994); Gordon et al., Drug Metab. Dispos., Vol.15, p.589 (1987); Wade D, Chem. Biol. Interact. Vol. 117, p. 191 (1991)).

Deuterium (2.01355 amu) is a stable and non-radioactive isotope of hydrogen with an atomic mass that is double that of hydrogen (1.0078 amu). The deuterium atom contains one proton and one neutron in the nucleus and has a natural abundance of 0.015%. Replacement of a hydrogen atom with deuterium may often result in a change in the rate of a chemical reaction. This phenomenon is known as the Kinetic Isotope Effect ("KIE"). For example, if a C-H bond is broken during a rate-determining step in a chemical reaction (*i.e.,* the step with the highest transition state energy), substitution of a deuterium for that hydrogen may cause a decrease in the reaction rate. This phenomenon is known as the Deuterium Kinetic Isotope Effect ("DKIE"). (*See, e.g.,* Foster et al., Adv. Drug Res., Vol. 14, pp. 1-36 (1985); Kushner et al., Can. J. Physiol. Pharmacol., Vol. 77, pp. 79-88 (1999)).

The magnitude of the DKIE can be expressed as the ratio between the rates of a given reaction in which a C-H bond is broken, and the same reaction where deuterium is substituted for hydrogen. The DKIE can range from about one (*i.e.,* no isotope effect) to very large numbers, such as fifty or more. Without being limited by a particular theory, a chemical reaction involving a therapeutic compound in which one or more hydrogen atoms have been replaced with deuterium may be much slower and thus affect the PK, PD and toxicity profiles of therapeutic compounds. Accordingly, there remains a need for enhancing the pharmaceutical properties of compounds by providing isotope-enriched analogues of 1,2,4-oxadiazole benzoic acid compounds and compositions thereof for treating or preventing a nonsense mutation mediated disease.

WO 2016/073545 describes phenyloxadiazole benzoic acids that can be administered as an inhibitor of premature translation termination and/or nonsense-mediated mRNA decay.

US 2004/204461 describes 1,2,4-oxadiazole benzoic acid compounds for treating or preventing diseases associated with nonsense mutations of mRNA.

### 3. SUMMARY

All subject matter not falling into the claims, being explicitly or implicitly stated as not forming part of the invention or being "described", is only for reference purposes an does not form part of the invention, which is exclusively defined by the claims.

Described herein are compounds of formula **I**: 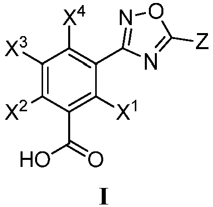 and pharmaceutically acceptable salts, hydrates, solvates, clathrates, prodrugs, polymorphs, stereoisomers, including enantiomers, diastereomers, racemates and mixtures of stereoisomers, thereof, wherein:
Z is substituted or unsubstituted aryl, substituted or unsubstituted heteroaryl, substituted or unsubstituted cycloalkyl, substituted or unsubstituted alkyl, substituted or unsubstitued alkenyl, substituted or unsubstituted heterocycle, substituted or unsubstituted arylalkyl, or aryloxyalkyl;
X¹, X², X³, and X⁴ are independently H, Deuterium ("D"), or Tritium ("T"); and
wherein one or more H atoms are replaced with D or T.

In a particular example Z is substituted aryl.

In another example Z is halo substituted aryl.

In another example Z is fluoro substituted aryl.

In another example Z is substituted phenyl.

In another example Z is halo substituted phenyl.

In another example Z is fluoro substituted phenyl.

In one embodiment, provided herein are compounds of formula **II**: 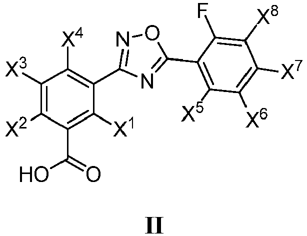 and pharmaceutically acceptable salts, hydrates, solvates, clathrates, polymorphs, stereoisomers, including enantiomers, diastereomers, racemates and mixtures of stereoisomers, thereof, wherein:
X¹, X², X³, X⁴ are H,

and wherein X⁵, X⁶, X⁷ and X⁸ are D

Also described herein are compounds of formula **II** wherein at least one of X¹, X², X³ and X⁴ is D or T.

In another example X⁵, X⁶, X⁷ and X⁸ is H and at least one of X¹, X², X³ and X⁴ is D or T.

Further described herein are methods of treating or preventing a disease ameliorated by modulation of premature translation termination, or ameliorating one or more symptoms associated therewith, comprising administering to a patient in need thereof a therapeutically effective amount of a compound of formula **I** and **II** or a pharmaceutically acceptable salt, hydrate, solvate, clathrate, prodrug, polymorph, stereoisomer, including enantiomers, diastereomers, racemates and mixtures of stereoisomers, thereof.

Described herein are methods of treating or preventing, or ameliorating a genetic or somatic nonsense mutation mediated disease, or one or more symptoms associated with or manifestations of a genetic or somatic nonsense mutation mediated disease, comprising administering to a patient in need thereof a therapeutically effective amount of a compound of formula **I** and **II** or a pharmaceutically acceptable salt, hydrate, solvate, clathrate, prodrug, polymorph, stereoisomer, including enantiomers, diastereomers, racemates and mixtures of stereoisomers, thereof.

Without being limited to any particular theory, the ability of the compounds of formula **I** and **II** to promote readthrough of premature stop codons makes them useful in the treatment or prevention of any disease which is caused in whole or in part by a nonsense mutation in DNA that produces a premature stop codon in the mRNA transcript. Examples of such diseases are described herein, and are well known to those skilled in the art, such as those skilled in the art of nonsense mutation mediated diseases. Such diseases can occur due to the decreased amount of functional protein produced as a result of premature termination of translation. Without being limited to any particular theory, the compounds of formula **I** and **II** allow the translation of mRNA to continue past the premature stop codon, resulting in the production of a functional protein. A powerful aspect described herein is that the therapeutic activity of compounds of formula **I** and **II** are not necessarily disease specific, instead are effective at treating of preventing any disease associated with a nonsense mutation. Further, the invention provided herein may be patient specific. That is, a patient may be screened to determine if this disease is associated with a nonsense mutation. If so, they can be treated with a compound of formula **I** or **II**.

In another embodiment provided herein is the compound provided herein or a pharmaceutical composition thereof for use in a method for treating a disease or disorder associated with a premature stop codon in a patient, wherein the disease or disorder associated with a premature stop codon is a cancer, a muscular dystrophy, a lysosomal storage disease, an autoimmune disease, a blood disease, a collagen disease, diabetes, a neurodegenerative disease, a proliferative disease, a cardiovascular disease, a pulmonary disease, an inflammatory disease, a central nervous system disease, an ocular disease, a metabolic disorder, a skin disease, a neuropathic disease, or a neuromuscular disorder.

The invention provided herein encompass the *in vitro* or *in vivo* use of a small molecule compound of formula II provided herein and the incorporation of a compound of formula **II** provided herein into pharmaceutical compositions and orally deliverable dosage forms useful in the treatment and prevention of a variety of diseases and disorders. Specific diseases and disorders include those ameliorated by the suppression of a premature stop codon in messenger RNA.

Pharmaceutical compositions, including dosage forms provided herein, which comprise a compound of formula **II** provided herein or a pharmaceutically acceptable salt, hydrate, solvate, clathrate, polymorph, stereoisomers, including enantiomers, diastereomers, racemates and mixtures of stereoisomers, thereof, can be used in the invention provided herein.

### 3.1 DEFINITIONS

As used herein, unless otherwise specified, the term "alkyl" means a saturated straight chain or branched non-cyclic hydrocarbon having from 1 to 20 carbon atoms, preferably 1-10 carbon atoms and most preferably 1-4 carbon atoms. An alkyl group can be unsubstituted or substituted where allowed by available valences.

As used herein, unless otherwise specified the term "alkenyl group" means a straight chain or branched non-cyclic hydrocarbon having from 2 to 20 carbon atoms, more preferably 2-10 carbon atoms, most preferably 2-6 carbon atoms, and including at least one carbon-carbon double bond. The double bond of an alkenyl group can be unconjugated or conjugated to another unsaturated group. An alkenyl group can be unsubstituted or substituted where allowed by available valences.

As used herein, unless otherwise specified the term "aryl" means a carbocyclic aromatic ring containing from 5 to 14 ring atoms. The ring atoms of a carbocyclic aryl group are all carbon atoms. Aryl ring structures include compounds having one or more ring structures such as mono-, bi-, or tricyclic compounds as well as benzo-fused carbocyclic moieties such as 5,6,7,8-tetrahydronaphthyl, and the like. Preferably, the aryl group is a monocyclic ring or bicyclic ring. Examples of aryl include phenyl, tolyl, anthracenyl, fluorenyl, naphthalene, indenyl, azulenyl, phenanthrenyl, and naphthyl. A carbocyclic aryl group can be unsubstituted or substituted where allowed by available valences.

As used herein, unless otherwise specified the term "heteroaryl" means a carbocyclic aromatic ring containing from 5 to 14 ring atoms and the ring atoms contain at least one heteroatom, preferably 1 to 3 heteroatoms, independently selected from nitrogen, oxygen, or sulfur. Heteroaryl ring structures include compounds having one or more ring structures such as mono-, bi-, or tricyclic compounds as well as fused heterocycle moities. Examples of heteroaryl include triazolyl, tetrazolyl, oxadiazolyl, pyridyl, furyl, benzofuranyl, thiophenyl, benzothiophenyl, benzoisoxazolyl, benzoisothiazolyl, quinolinyl, pyrrolyl, indolyl, oxazolyl, benzoxazolyl, imidazolyl, benzimidazolyl, thiazolyl, benzothiazolyl, isoxazolyl, pyrazolyl, isothiazolyl, pyridazinyl, pyrimidinyl, pyrazinyl, triazinyl, cinnolinyl, phthalazinyl, quinazolinyl, benzoquinazolinyl, acridinyl, pyrimidyl, and oxazolyl. A group can be unsubstituted or substituted where allowed by available valences.

As used herein, unless otherwise specified the term "cycloalkyl" means a monocyclic or polycyclic saturated ring comprising carbon and hydrogen atoms and having no carbon-carbon multiple bonds. A cycloalkyl group can be unsubstituted or substituted. Examples of cycloalkyl include, but are not limited to, (C₃-C₇)cycloalkyl groups, including cyclopropyl, cyclobutyl, cyclopentyl, cyclohexyl, and cycloheptyl, and saturated cyclic and bicyclic terpenes. A cycloalkyl group can be unsubstituted or substituted where allowed by available valences. Preferably, the cycloalkyl group is a monocyclic ring or bicyclic ring.

As used herein, unless otherwise specified the term "heterocycle" means a monocyclic or polycyclic ring comprising carbon and hydrogen atoms, optionally having 1 to 4 multiple bonds, and the ring atoms contain at least one heteroatom, preferably 1 to 3 heteroatoms, independently selected from nitrogen, oxygen, and sulfur. Heterocyclyl ring structures include compounds having one or more ring structures such as mono-, bi-, or tricylic compounds. Preferably, the heterocyclic group is a monocyclic ring or bicyclic ring. Examples of heterocyclyl include, but are not limited to morpholinyl, pyrrolidinonyl, pyrrolidinyl, piperidinyl, hydantoinyl, valerolactamyl, oxiranyl, oxetanyl, tetrahydrofuranyl, tetrahydropyranyl, tetrahydropyridinyl, tetrahydroprimidinyl, tetrahydrothiophenyl, tetrahydrothiopyranyl, and the like. A heterocyclyl ring can be unsubstituted or substituted where allowed by available valences.

As used herein, unless otherwise specified the term "arylalkyl" means -(alkyl)-(aryl), wherein alkyl and aryl are defined above, including, but not limited to -(CH₂)phenyl, - (CH₂)₂phenyl, -(CH₂)₃phenyl, -CH(phenyl)₂, -CH(phenyl)₃, -(CH₂)tolyl, -(CH₂)anthracenyl, - (CH₂)fluorenyl, -(CH₂)indenyl, -(CH₂)azulenyl, -(CH₂)naphthyl, and the like.

The term "aryloxyalkyl," as used herein, refers to an aryl group, as defined herein, appended to the parent molecular moiety through a "-O-alkyl" group, with alkyl as defined herein.

As used herein, unless otherwise specified the term "halogen" or "halo" means fluorine, chlorine, bromine, or iodine.

As used herein, unless otherwise specified, the term "substituted" means a group may be substituted by one or more independent substituents, examples of which include, but are not limited to, halo, alkyl, alkoxy, trifluoromethyl, trifluoromethoxy, hydroxy, alkoxy, cycloalkyoxy, heterocylooxy, oxo, alkanoyl, alkylcarbonyl, cycloalkyl, aryl, aryloxy, aralkyl, alkanoyloxy, cyano, azido, amino, alkylamino, -S(O)₂OH, arylamino, aralkylamino, cycloalkylamino, heterocycloamino, mono and disubstituted amino in which the two substituents on the amino group are selected from alkyl, aryl, aralkyl, alkanoylamino, aroylamino, aralkanoylamino, substituted alkanoylamino, substituted arylamino, substituted aralkanoylamino, thiol, alkylthio, arylthio, aralkylthio, cycloalkylthio, heterocyclothio, alkylthiono, arylthiono, aralkylthiono, alkylsulfonyl, arylsulfonyl, aralkylsulfonyl, oxygen, sulfonamido (*e.g.,* SO₂NH₂), substituted sulfonamido, nitro, carboxy, carbamyl (*e.g.,* CONH₂), substituted carbamyl (*e.g*., CONH alkyl, CONH aryl, CONH aralkyl or instances where there are two substituents on the nitrogen selected from alkyl, aryl or aralkyl), alkoxycarbonyl, aryl, substituted aryl, guanidino and heterocyclo, such as indolyl, imidazolyl, furyl, thienyl, thiazolyl, pyrrolidyl, pyridyl, pyrimidyl, and the like. Wherein, as noted above, the substituents themselves are further substituted, such further substituents are selected from the group consisting of halogen, alkyl, alkoxy, aryl, and aralkyl. In one example the substituent is D or T. In another example, the substituent may be isotopically enriched with D or T.

As used herein, "premature translation termination" refers to the result of a mutation that changes a codon corresponding to an amino acid to a stop codon.

As used herein, a "premature termination codon" or "premature stop codon" refers to the occurrence of a stop codon where a codon corresponding to an amino acid should be.

As used herein, a "nonsense mutation" is a point mutation changing a codon corresponding to an amino acid to a stop codon.

As used herein, "nonsense suppression" refers to the inhibition or suppression of premature translation.

As used herein, "modulation of premature translation termination" refers to the regulation of gene expression by altering the level of nonsense suppression. For example, if it is desirable to increase production of a functional protein instead of the defective protein encoded by a gene having a nonsense mutation (*i.e.,* to permit readthrough of the premature stop codon in the mRNA expressed by the disease gene so translation of the protein can occur), then modulation of premature translation termination entails up-regulation of nonsense suppression. Conversely, if it is desirable to promote the degradation of an mRNA with a premature stop codon, then modulation of premature translation termination entails down-regulation of nonsense suppression.

As used herein, the term "patient" means a non-primate animal (*e.g*., cow, horse, sheep, pig, chicken, turkey, quail, cat, dog, mouse, rat, rabbit, guinea pig, etc.), preferably a mammal such as a non-primate or a primate (*e.g*., monkey and human), most preferably a human. In certain embodiments, the patient is an infant, child, adolescent or adult. In one embodiment, it has been determined through pre-screening that the patient possesses a nonsense mutation. In another embodiment, pre-screening has determined which nonsense mutation the patient has (*i.e.,* UAA, UGA, or UAG). In another embodiment, the patient is infected with bacterial cells (*e.g., Pseudomonas aeruginosa*)*.* In another embodiment, the cells of the patient are virally infected.

As used herein, a "therapeutically effective amount" refers to that amount of a compound of formula **I** or **II** sufficient to provide a therapeutic benefit in the treatment or management of the disease or to delay or minimize symptoms associated with the disease. Further, a therapeutically effective amount with respect to a compound of formula **I** or **II** means that amount of therapeutic agent alone, or in combination with other therapies, that provides a therapeutic benefit in the treatment or management of the disease. Used in connection with an amount of a compound of formula **I** or **II**, the term can encompass an amount that improves overall therapy, reduces or avoids symptoms or causes of disease, or enhances the therapeutic efficacy of or synergies with another therapeutic agent.

As used herein, a "therapeutic protocol" refers to a regimen of timing and dosing of one or more therapeutic agents.

A used herein, a "protocol" includes dosing schedules and dosing regimens.

As used herein, the terms "prevent", "preventing", and "prevention" refer to the prevention of the onset recurrence, spread or of the disease in a subject resulting from the administration of a therapeutic agent.

As used herein, the terms "treat", "treating", and "treatment" refer to the eradication or amelioration of the disease or symptoms associated with the disease. In certain embodiments, such terms refer to minimizing the spread or worsening of the disease resulting from the administration of one or more therapeutic agents to a subject with such a disease.

As used herein, the term "pharmaceutically acceptable salts" refer to salts prepared from pharmaceutically acceptable non-toxic acids or bases including inorganic acids and bases and organic acids and bases. Suitable pharmaceutically acceptable base addition salts for the compound of formula **I** or **II** include, but are not limited to, metallic salts made from aluminum, calcium, lithium, magnesium, potassium, sodium and zinc or organic salts made from lysine, N,N'-dibenzylethylenediamine, chloroprocaine, choline, diethanolamine, ethylenediamine, meglumine (N-methylglucamine), tromethamine, L-lysine, L-arginine, L-histidine, and procaine. Suitable non-toxic acids include, but are not limited to, inorganic and organic acids such as acetic, alginic, anthranilic, benzenesulfonic, benzoic, bromic, camphorsulfonic, chloric, citric, ethenesulfonic, formic, fumaric, furoic, galacturonic, gluconic, glucuronic, glutamic, glycolic, hydrobromic, hydrochloric, isethionic, lactic, maleic, malic, mandelic, methanesulfonic, mucic, nitric, pamoic, pantothenic, phenylacetic, phosphoric, propionic, salicylic, stearic, succinic, sulfanilic, sulfonic, sulfuric, tartaric acid, and p-toluenesulfonic acid. Specific non-toxic acids include hydrochloric, hydrobromic, phosphoric, sulfuric, and methanesulfonic acids. Examples of specific salts thus include a magnesium salt, a potassium salt, a sodium salt, a tromethamine salt, an L-lysine salt, an L-arginine salt, an N-methyl glucamine salt and an L-histidine salt. Other examples of salts are well known in the art, *see, e.g.,* Remington's Pharmaceutical Sciences, 18th ed., Mack Publishing, Easton PA (1995).

As used herein and unless otherwise indicated, the term "optically pure" or "stereomerically pure" means a stereoisomer of a compound is substantially free of the other stereoisomers of that compound. For example, a stereomerically pure compound having one chiral center will be substantially free of the opposite enantiomer of the compound. A stereomerically pure compound having two chiral centers will be substantially free of other diastereomers of the compound. A typical stereomerically pure compound comprises greater than about 80% by weight of one stereoisomer of the compound and less than about 20% by weight of other stereoisomers of the compound, more preferably greater than about 90% by weight of one stereoisomer of the compound and less than about 10% by weight of the other stereoisomers of the compound, even more preferably greater than about 95% by weight of one stereoisomer of the compound and less than about 5% by weight of the other stereoisomers of the compound, more preferably greater than about 97% by weight of one stereoisomer of the compound and less than about 3% by weight of the other stereoisomers of the compound, more preferably greater than about 98% by weight of one stereoisomer of the compound and less than about 2% by weight of the other stereoisomers of the compound, more preferably greater than about 99% by weight of one stereoisomer of the compound and less than about 1% by weight of the other stereoisomers of the compound, and more preferably greater than about 99.5% by weight of one stereoisomer of the compound and less than about 0.5% by weight of the other stereoisomers of the compound.

As used herein and unless otherwise indicated, the term "enantiomerically pure" means a stereomerically pure composition of a compound having one chiral center.

It should be noted that if there is a discrepancy between a depicted structure and a name given that structure, the depicted structure is to be accorded more weight. In addition, if the stereochemistry of a structure or a portion of a structure is not indicated with, for example, bold or dashed lines, the structure or portion of the structure is to be interpreted as encompassing all stereoisomers of it.

As used herein and unless otherwise indicated, the term "hydrogen isotope" includes deuterium ("D") and tritium ("T"). In one embodiment, the hydrogen isotope is D. In another example, the hydrogen isotope is T.

The term "isotopically enriched" refers to an atom of a specific position of a compound having an isotopic composition other than the natural isotopic composition of that atom. "Isotopically enriched" can also refer to a compound containing at least one atom having an isotopic composition other than the natural isotopic composition of that atom. As used herein, an "isotopologue" is an isotopically enriched compound.

The term "isotopic enrichment" refers to the percentage of incorporation of an amount of a specific isotope at a given atom in a molecule in the place of that atom's natural isotopic composition. For example, deuterium enrichment of 1% at a given position means that 1% of the molecules in a given sample contain deuterium at the specified position. Because the naturally occurring distribution of deuterium is about 0.0156%, deuterium enrichment at any position in a compound synthesized using non-enriched starting materials is about 0.0156%.

As used herein, a "deuterium" group is a stable isotope of hydrogen having one proton and one neutron.

As used herein, a "tritium" group is a stable isotope of hydrogen having one proton and two neutrons.

With regard to the compounds provided herein, when a particular atomic position is designated as having deuterium or "D," it is understood that the abundance of deuterium at that position is substantially greater than the natural abundance of deuterium, which is about 0.0156%.

With regard to the compounds provided herein, when a particular atomic position is designated as having tritium or "T," it is understood that the abundance of tritium at that position is substantially greater than the natural abundance of tritium, which is about 10⁻¹⁸%.

A position designated as having hydrogen isotopic-enrichment typically has a minimum isotopic enrichment factor of, in particular embodiments, at least 15% deuterium or tritium incorporation, at least 30% deuterium or tritium incorporation, at least 45% deuterium or tritium incorporation, at least 55% deuterium or tritium incorporation, at least 60% deuterium or tritium incorporation, at least 67.5% deuterium or tritium incorporation, at least 75% deuterium or tritium incorporation, at least 82.5% deuterium or tritium incorporation, at least 90% deuterium or tritium incorporation, at least 95% deuterium or tritium incorporation, at least 97% deuterium or tritium incorporation, at least 99% deuterium or tritium incorporation, or at least 99.5% deuterium or tritium incorporation at each designated hydrogen isotope-enriched atom.

It is understood that one or more hydrogen isotope atoms may exchange with hydrogen under physiological conditions.

The isotopic enrichment and isotopic enrichment factor of the compounds provided herein can be determined using conventional analytical methods known to one of ordinary skill in the art, including mass spectrometry and nuclear magnetic resonance spectroscopy.

### 4. DETAILED DESCRIPTION OF THE INVENTION

### 4.1 COMPOUNDS

All subject matter not falling into the claims, being explicitly or implicitly stated as not forming part of the invention or being "described", is only for reference purposes an does not form part of the invention, which is exclusively defined by the claims.

Described herein are compounds of formula **I**: 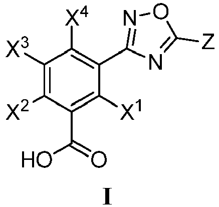 and pharmaceutically acceptable salts, hydrates, solvate, clathrates, prodrugs, polymorphs, stereoisomers, including enantiomers, diastereomers, racemates and mixtures of stereoisomers, thereof,
wherein Z is substituted or unsubstituted aryl, substituted or unsubstituted heteroaryl, substituted or unsubstituted cycloalkyl, substituted or unsubstituted alkyl, substituted or unsubstitued alkenyl, substituted or unsubstituted heterocycle, substituted or unsubstituted arylalkyl, or aryloxyalkyl;
X¹, X², X³, and X⁴ are independently H, D, or T; and
wherein one or more H atoms of formula **I** are replaced with D or T.

In a particular example Z is substituted aryl.

In another example Z is halo substituted aryl.

In another example Z is fluoro substituted aryl.

In another example Z is substituted phenyl.

In another example Z is halo substituted phenyl.

In another example Z is fluoro substituted phenyl.

In certain embodiments, provided herein are compounds of formula **II**: 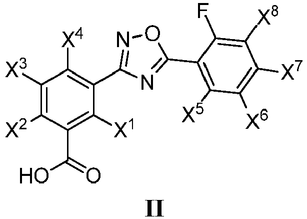 and pharmaceutically acceptable salts, hydrates, solvate, clathrates, polymorphs, stereoisomers, including enantiomers, diastereomers, racemates and mixtures of stereoisomers, thereof,
wherein X¹ X², X³, X⁴ are H and wherein X⁵, X⁶, X⁷ or X⁸ is D.

In an embodiment of the invention, a pharmaceutical composition comprising a compound of formula (II) and a pharmaceutically acceptable carrier, excipient or diluent is included.

A compound of formula (II) or its pharmaceutical composition for use in a method for treating a disease or disorder associated with a premature stop codon in a patient, wherein the disease or disorder associated with a premature stop codon is a cancer, a muscular dystrophy, a lysosomal storage disease, an autoimmune disease, a blood disease, a collagen disease, diabetes, a neurodegenerative disease, a proliferative disease, a cardiovascular disease, a pulmonary disease, an inflammatory disease, a central nervous system disease, an ocular disease, a metabolic disorder, a skin disease, a neuropathic disease, or a neuromuscular disorder is also included into the scope of the invention. Another embodiment of the appliction relates to the compound or the pharmaceutical composition for use , wherein the disease or disorder associated with a premature stop codon is muscular dystrophy; and wherein the muscular dystrophy is Duchenne muscular dystrophy.

Also described herein are compounds of formula **II** wherein at least one of X¹, X², X³ and X⁴ is D or T.

Also described are compounds where X⁵, X⁶, X⁷ and X⁸ is H and at least one of X¹, X², X³ and X⁴ is D or T.

In certain embodiments, the X⁵, X⁶, X⁷ and X⁸ atoms of a compound of formula **II** are hydrogen isotope-enriched. For example, compounds described include the following listed compounds of Tables 1, 2, and 3, wherein the label "H^{∗}" indicates a hydrogen isotope-enriched atomic position, *i.e.,* a sample comprising the given compound is hydrogen isotope-enriched at the indicated position(s) above the natural abundance of isotope, and any hydrogen atom not designated as a hydrogen isotope may be present according to natural abundance:

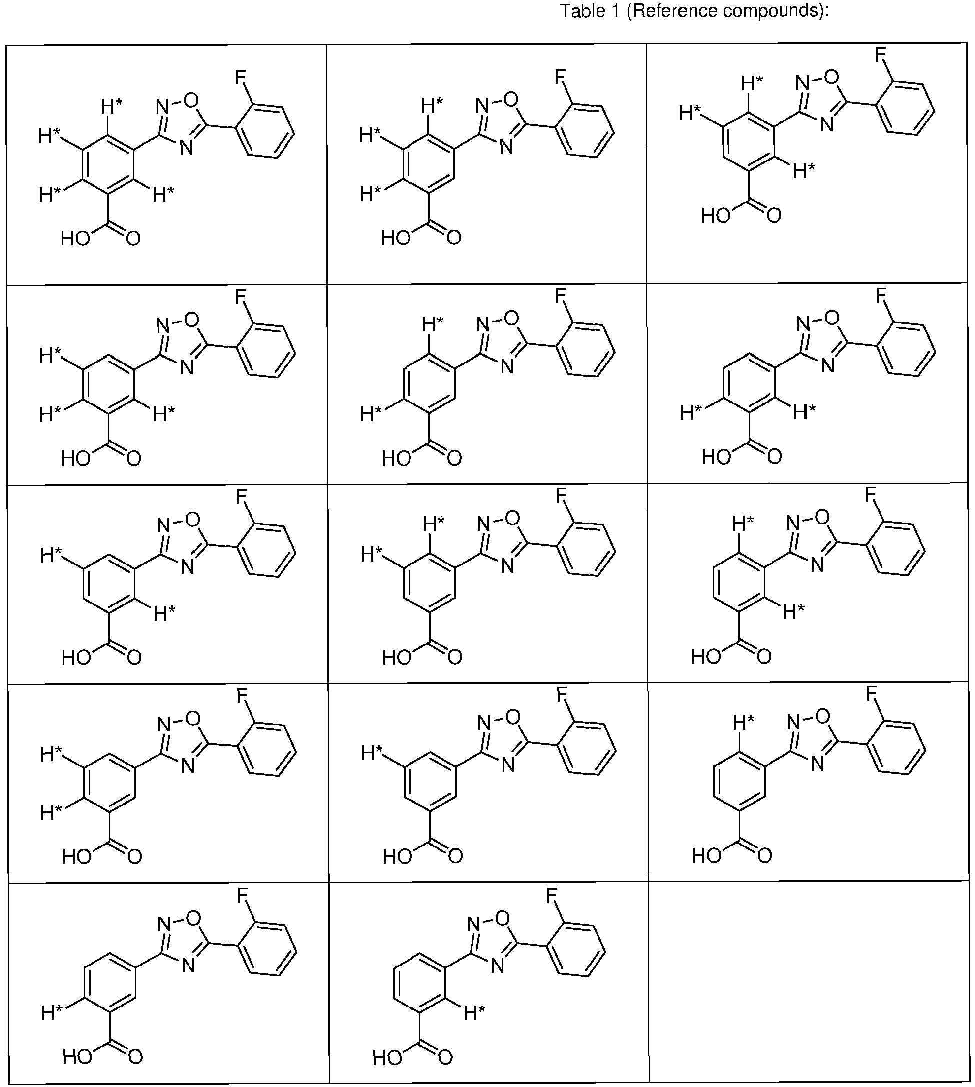

and pharmaceutically acceptable salts, hydrates, solvate, clathrates, prodrugs, polymorphs, stereoisomers, including enantiomers, diastereomers, racemates and mixtures of stereoisomers, thereof.

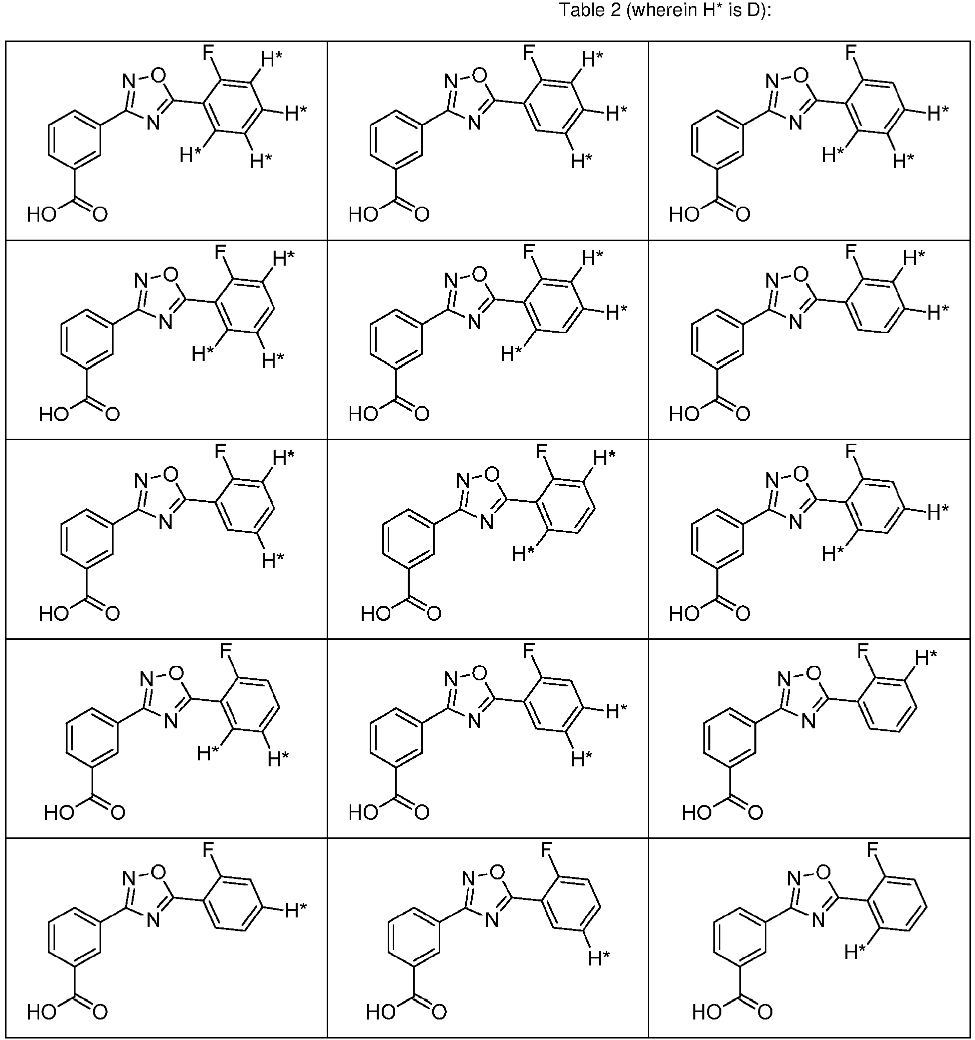

and pharmaceutically acceptable salts, hydrates, solvate, clathrates, polymorphs, stereoisomers, including enantiomers, diastereomers, racemates and mixtures of stereoisomers, thereof.

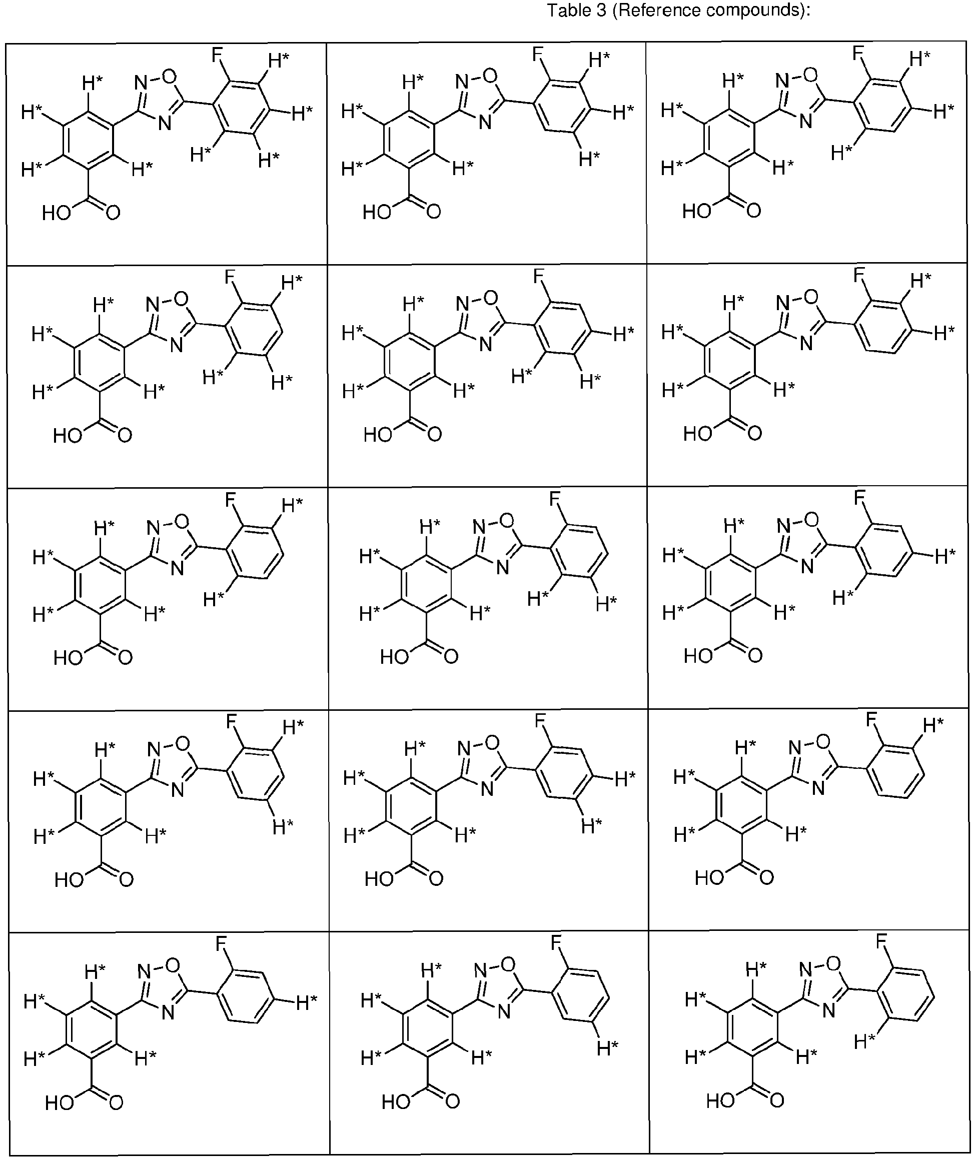

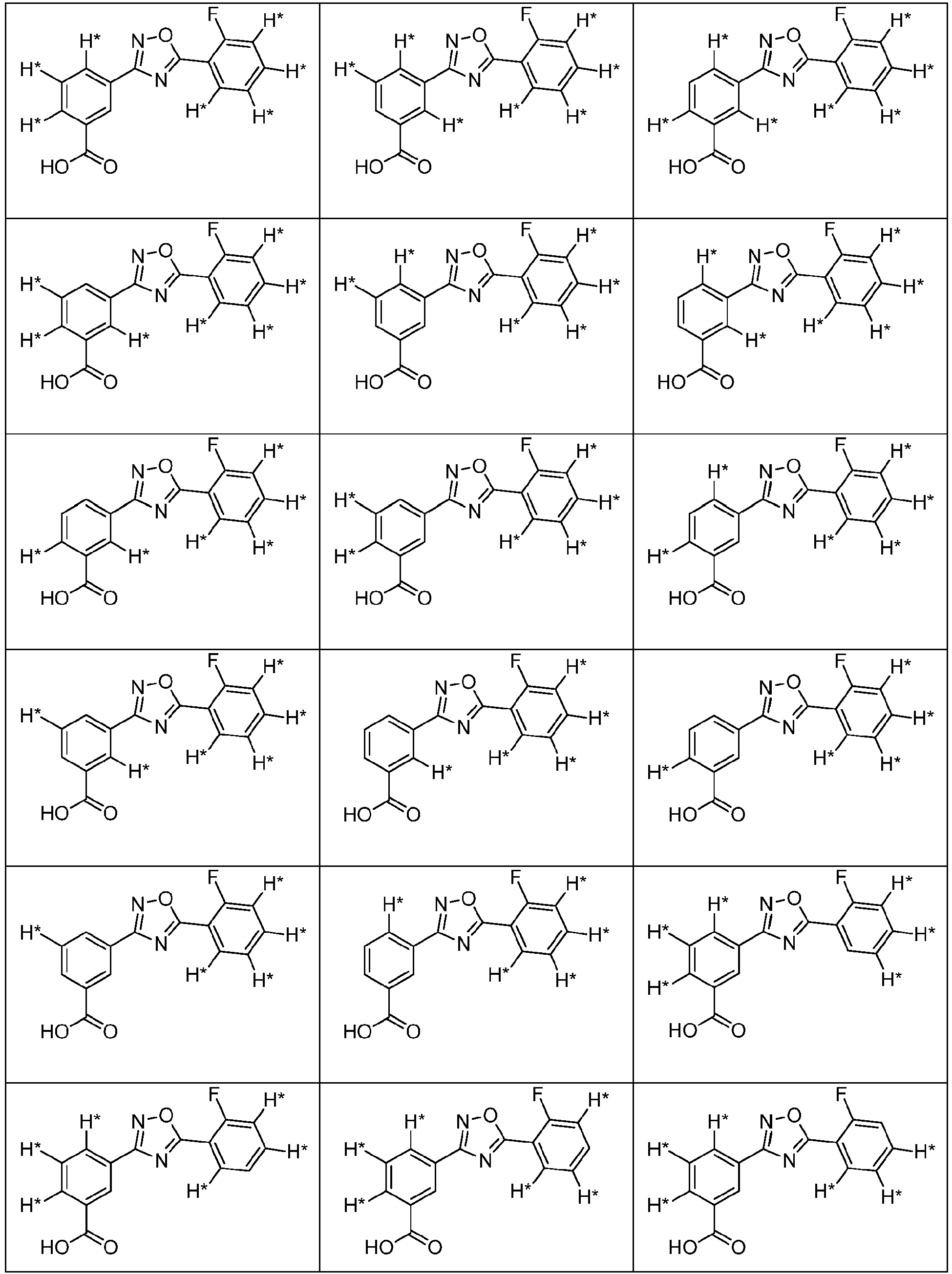

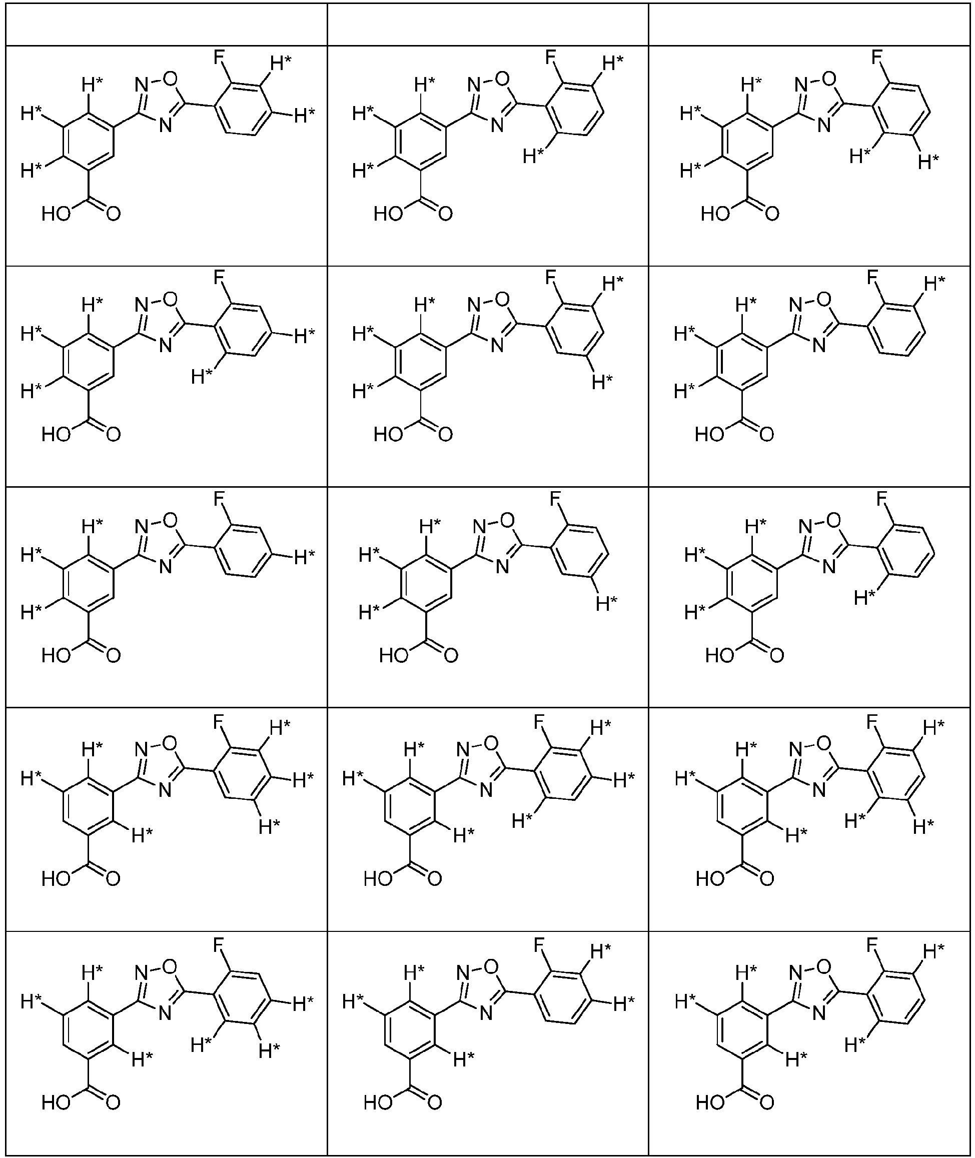

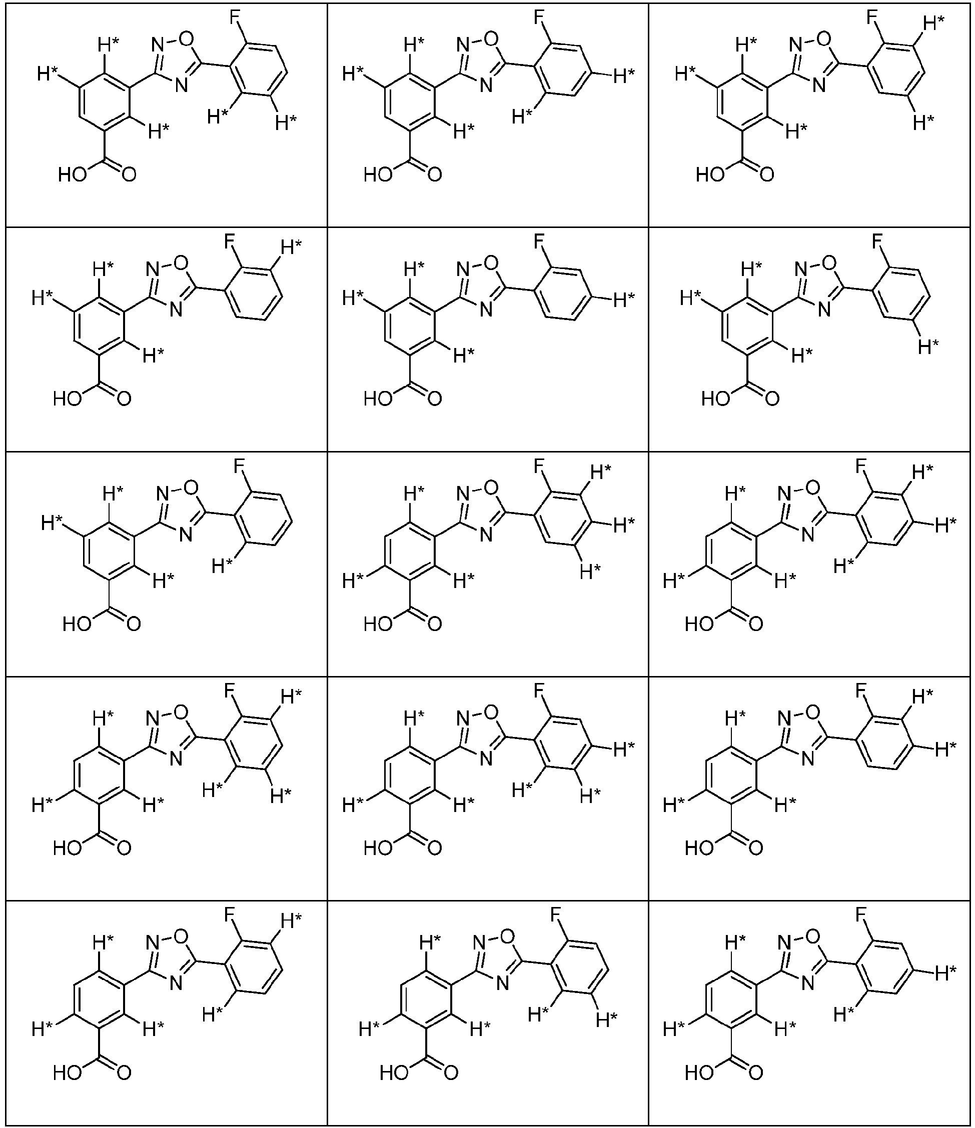

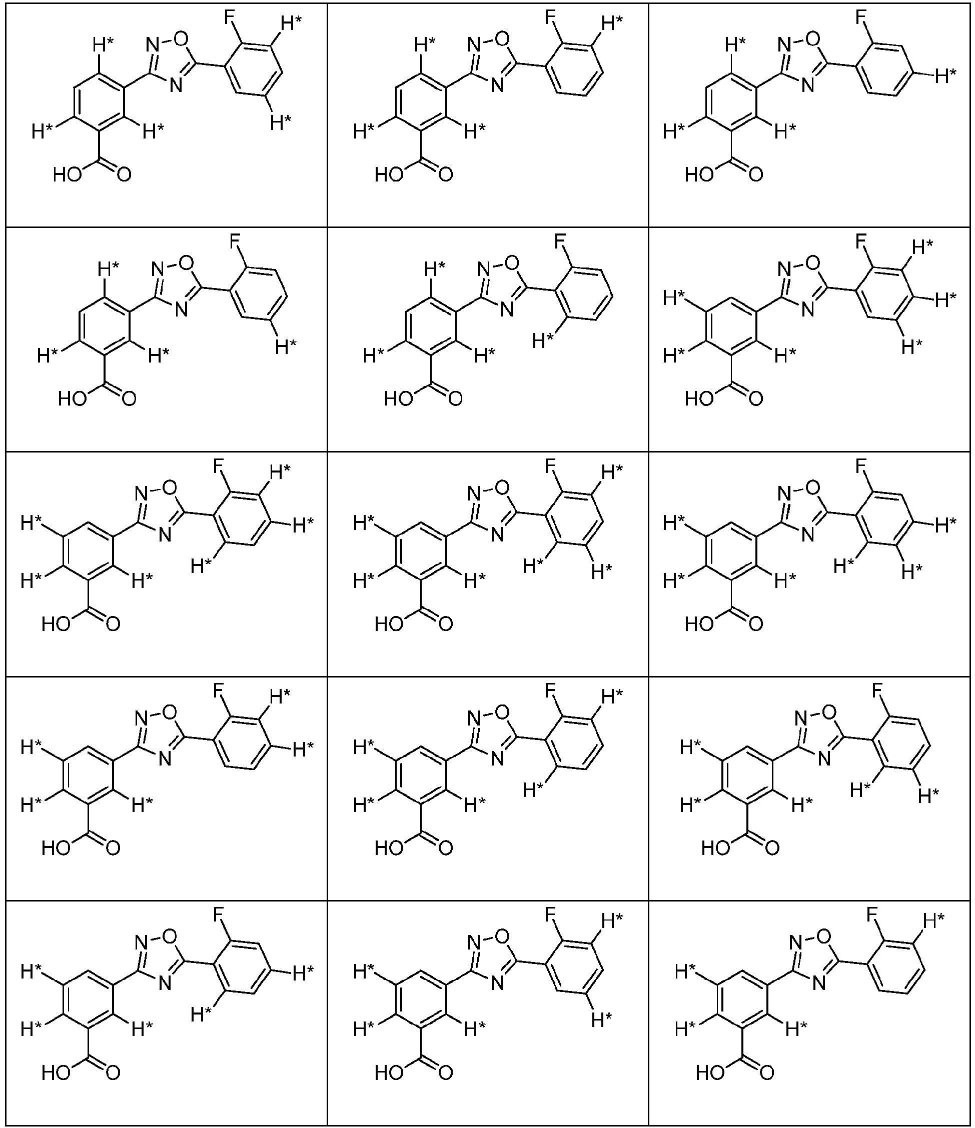

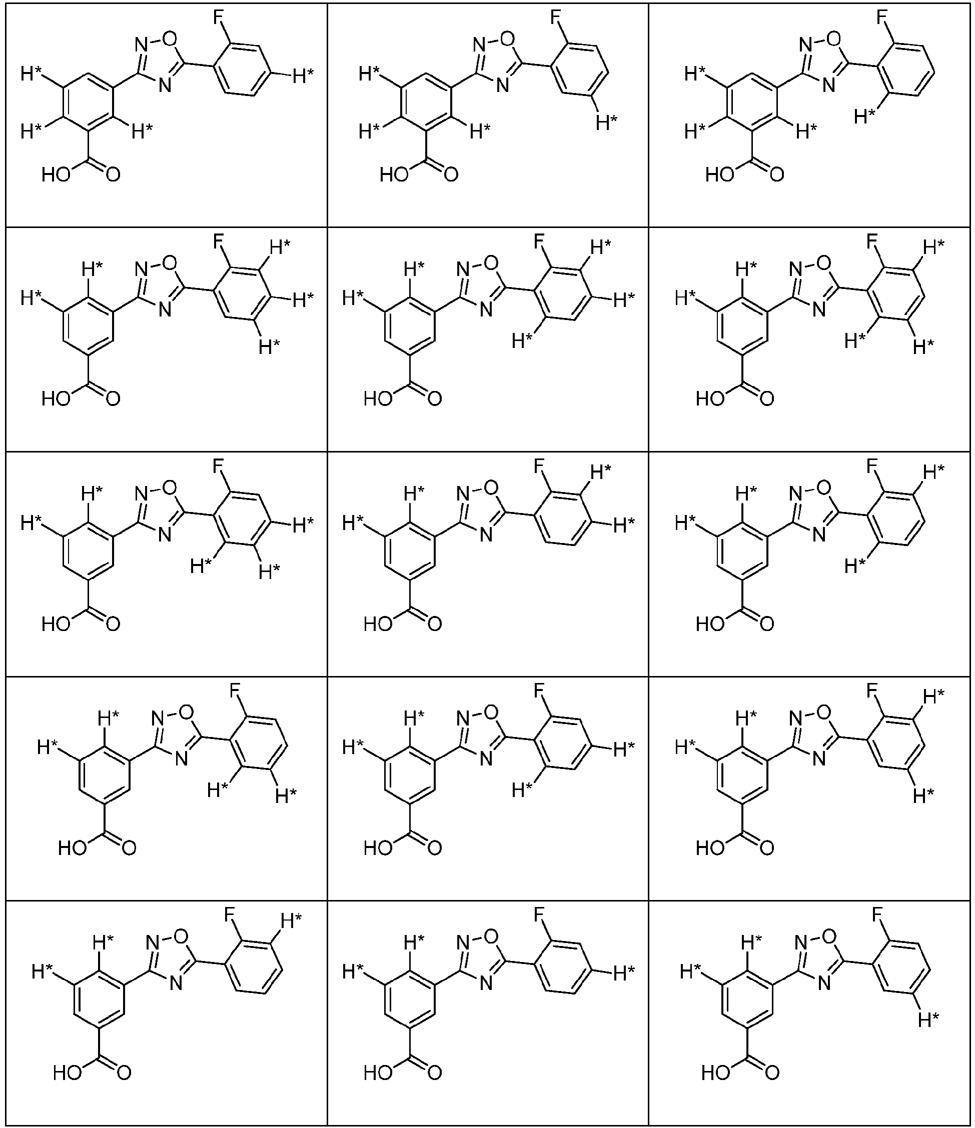

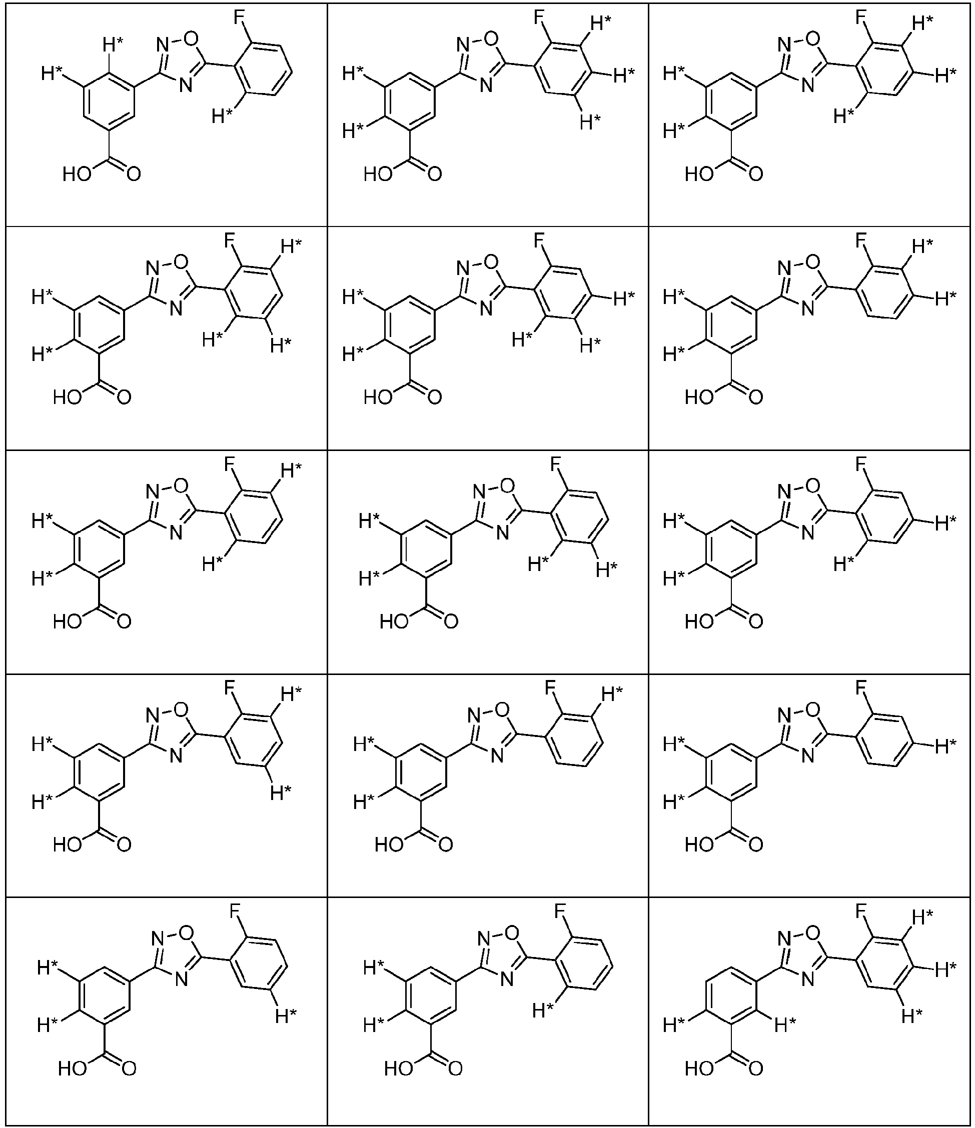

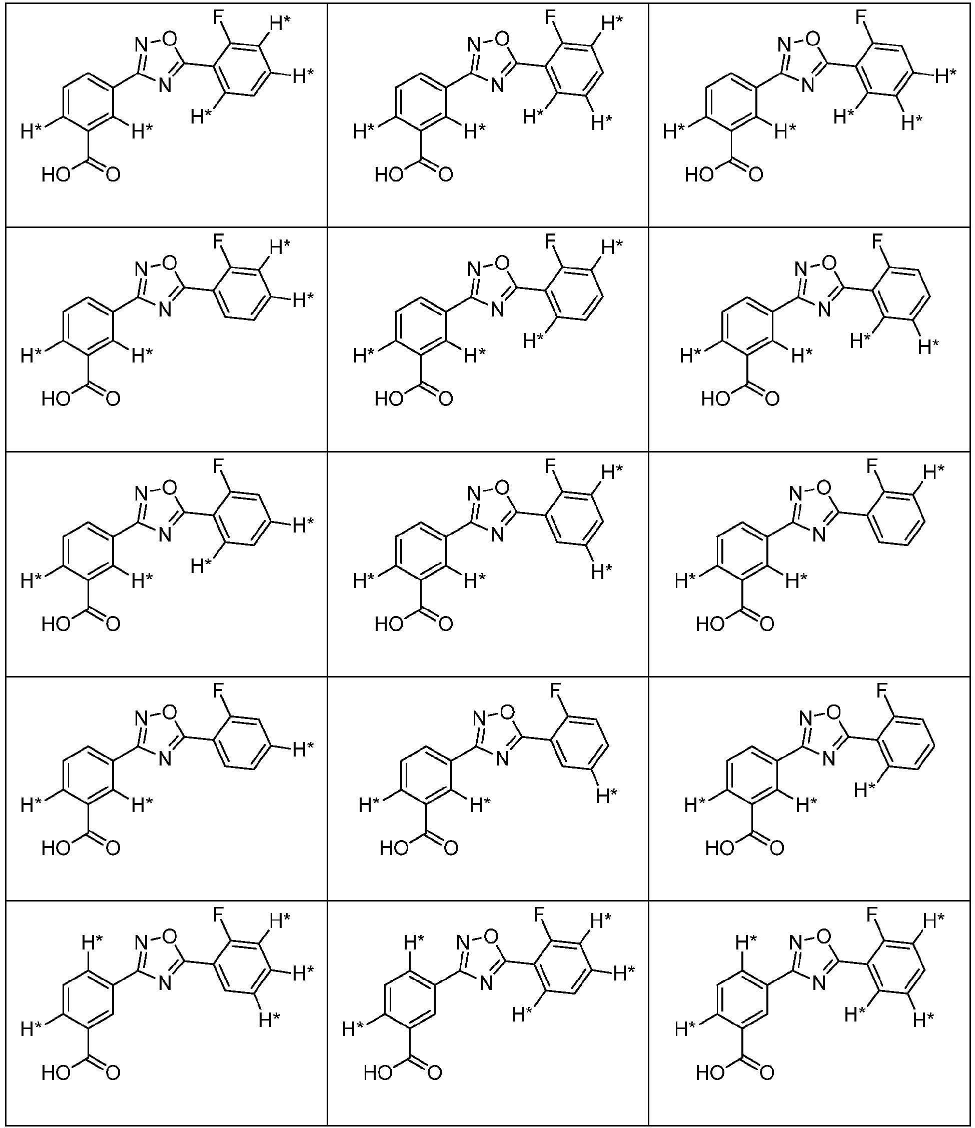

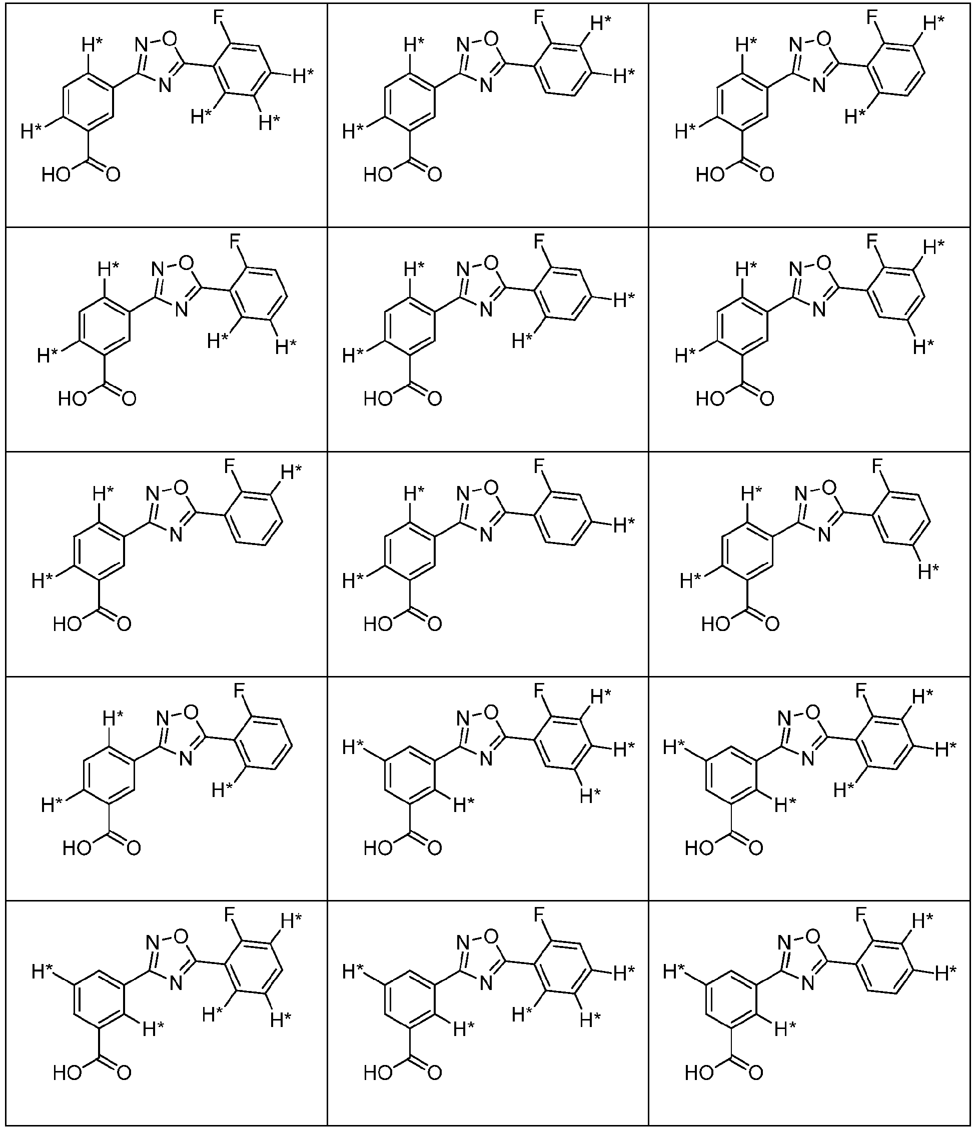

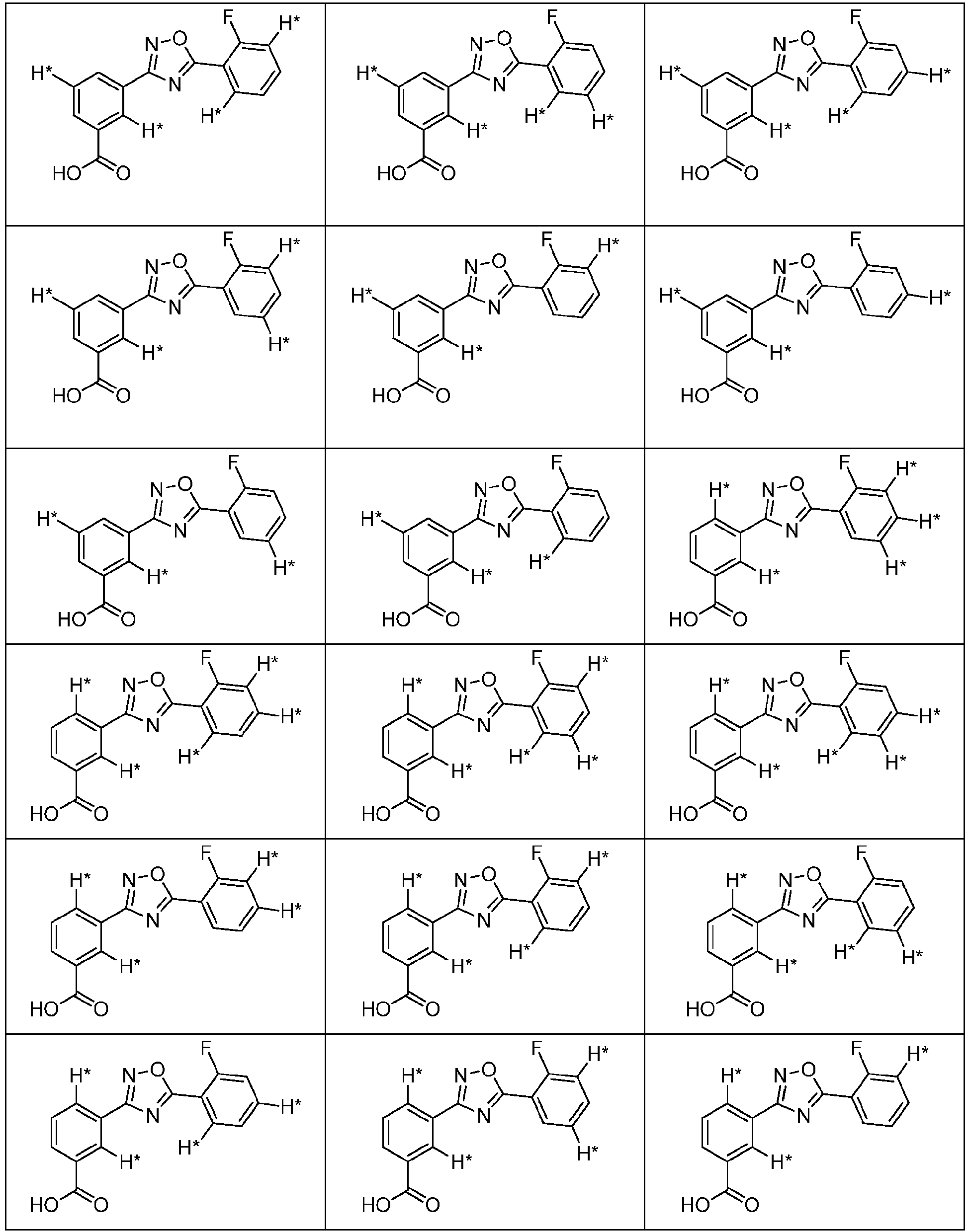

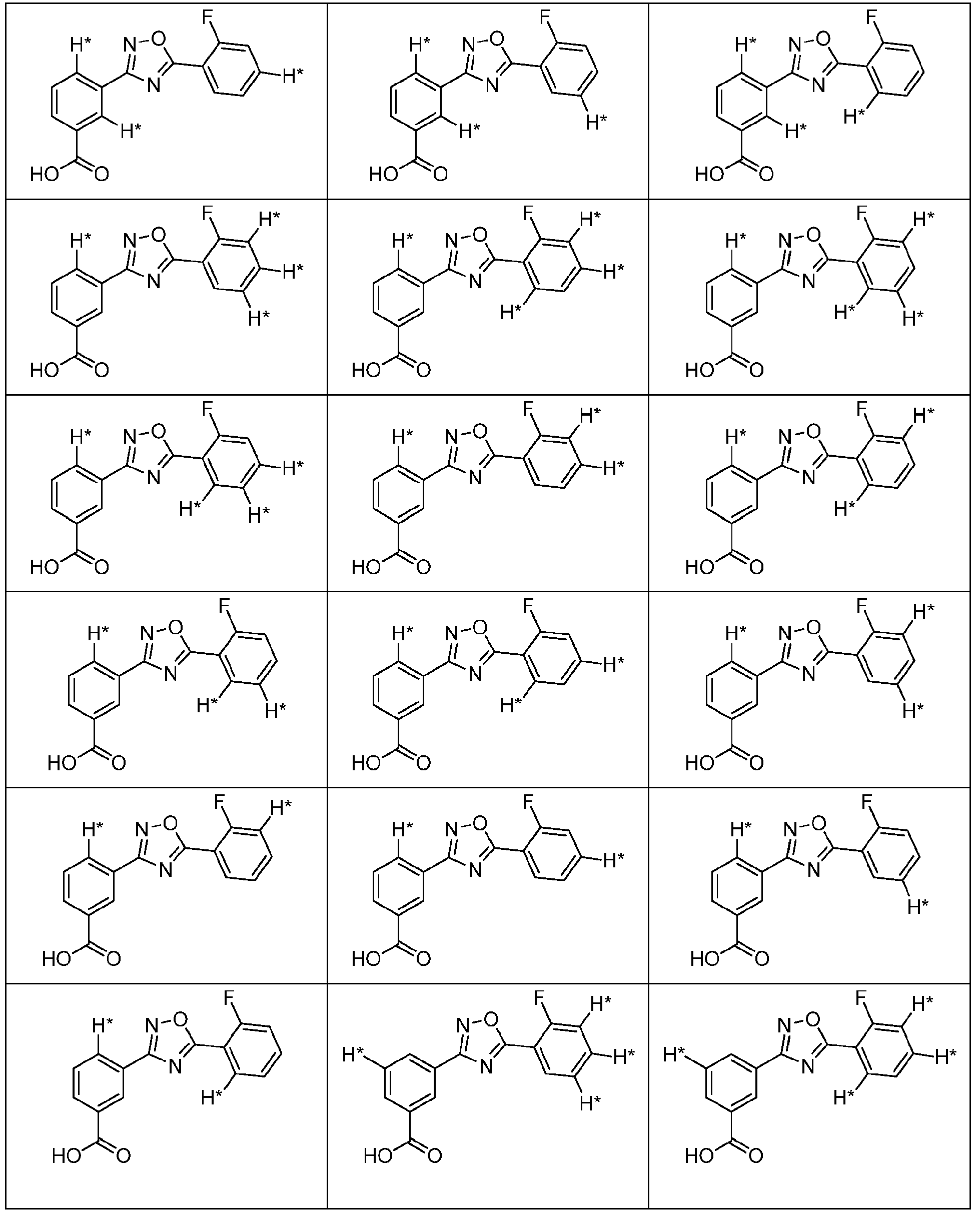

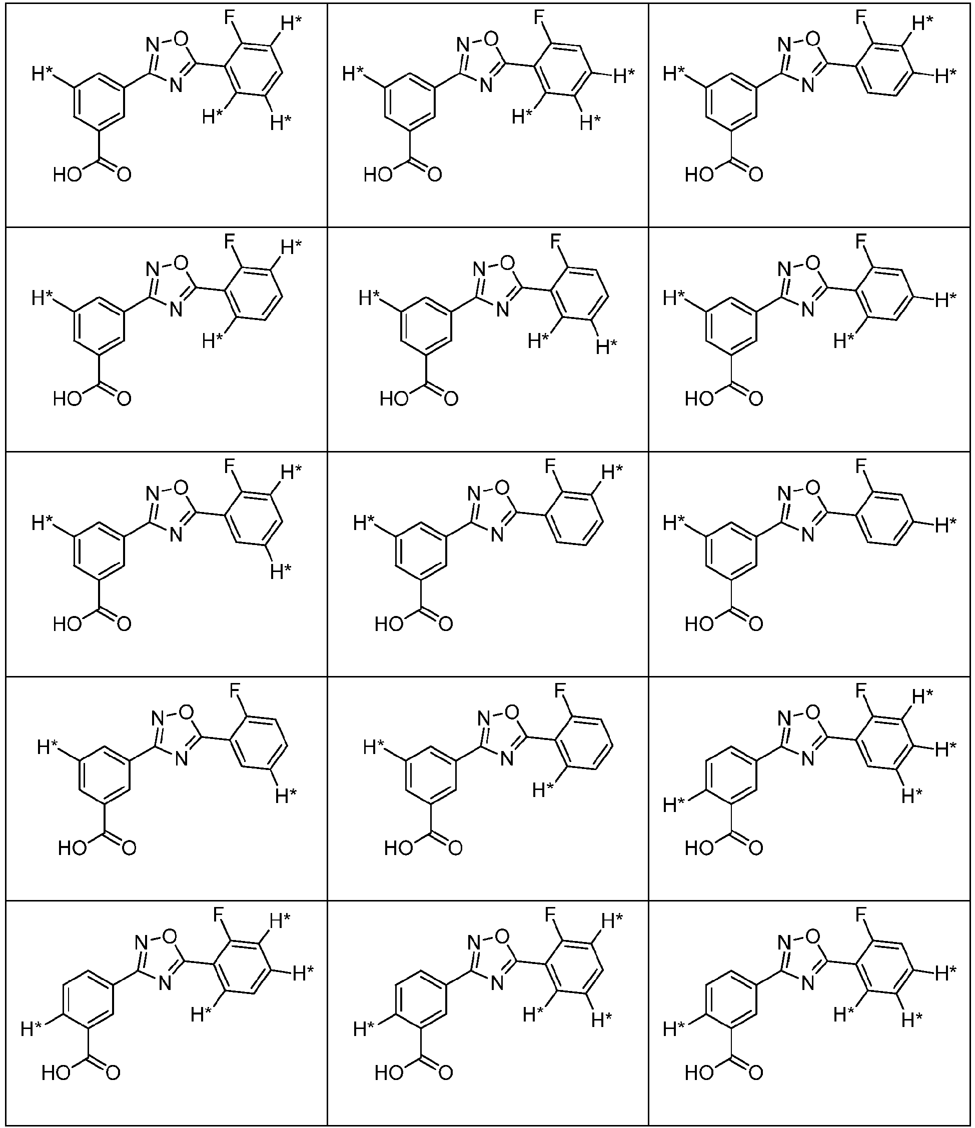

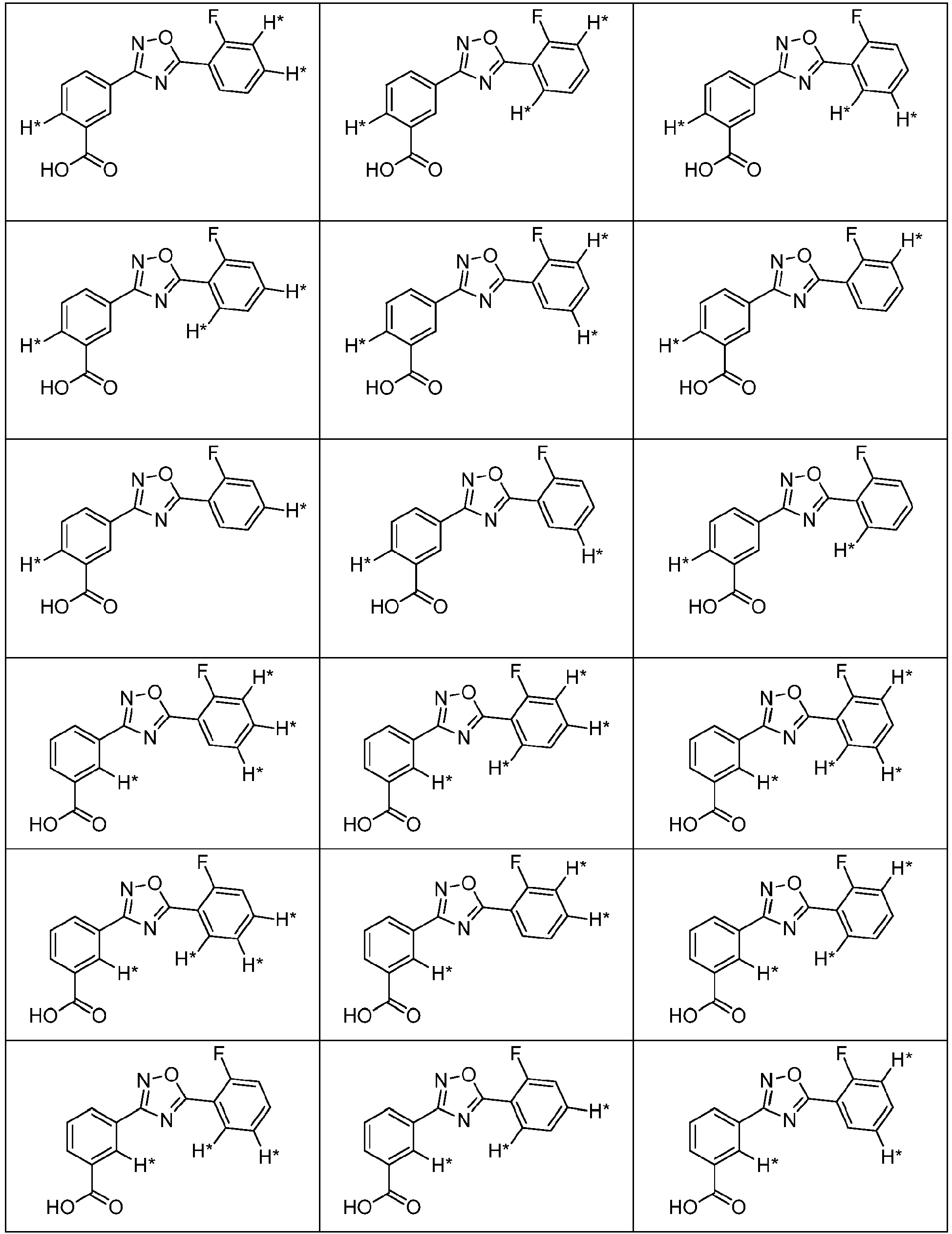

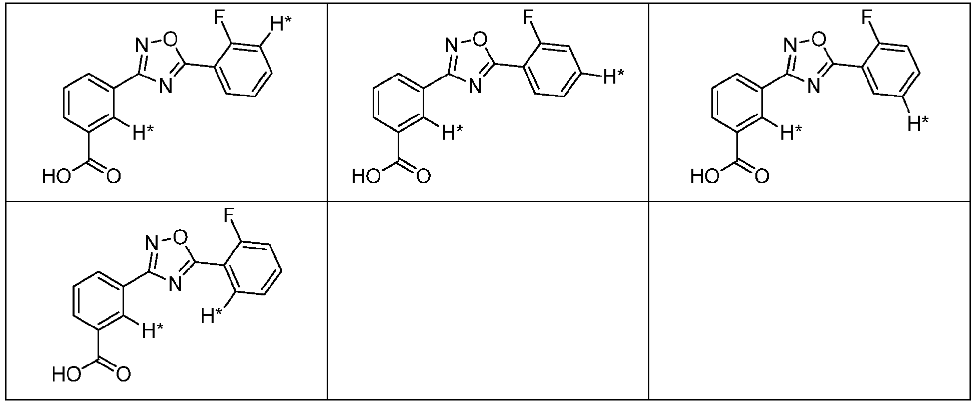

and pharmaceutically acceptable salts, hydrates, solvate, clathrates, prodrugs, polymorphs, stereoisomers, including enantiomers, diastereomers, racemates and mixtures of stereoisomers, thereof.

### 4.2 BIOLOGICAL ASSAYS AND ANIMAL STUDIES

Compounds that modulate premature translation termination can be identified by a number of techniques. For example, methods for screening compounds that modulate the post-transcriptional expression of any gene expressing a premature translation stop codon are described in International Patent Publication No. WO 01/44516 A2. In a preferred embodiment, a mRNA with a premature termination codon is translated *in vitro* and is used to screen a library of test compounds. In a preferred embodiment, the mRNA having a premature termination codon is a reporter gene having a premature termination codon.

Two assays were developed for use in high throughput screens to identify small molecules that promote nonsense suppression. Each assay utilized luciferase because it is a functional reporter gene assay (light is only produced if the protein is functional), and it is extremely sensitive (light intensity is proportional to luciferase concentration in the nM range). The first assay is a cell-based luciferase reporter assay and the second is a biochemical assay consisting of rabbit reticulocyte lysate and a nonsense-containing luciferase reporter mRNA. In the cell-based assay, a luciferase reporter construct containing a UGA premature termination codon was stably transfected in 293 Human Embryonic Kidney cells. In the biochemical assay, mRNA containing a UGA premature termination codon was used as a reporter in an *in vitro* translation reaction using rabbit reticulocyte lysate supplemented with tRNA, hemin, creatine kinase, amino acids, KOAc, Mg(OAc)₂, and creatine phosphate. Synthetic mRNA was prepared *in vitro* using the T7 promoter and the MegaScript *in vitro* transcription kit (Ambion). In both of the biochemical and cell-based assays, addition of a small molecule known to allow readthrough of premature termination codons, a compound of formula **I** or **II**, resulted in increased luciferase activity and was, therefore, used as an internal standard.

An example of a biological assay for mucopolysaccharidosis type VI ("MPS VI") includes, but is not limited to an MPS VI primary fibroblast based assay *(see, e.g.,* Bartolomeo et al., 2013 J Inherit Metab Dis, 36:363-371).

Examples of biological assays for heritable pulmonary arterial hypertension include, but are not limited to a pulmonary artery endothelial cell based assay, a pulmonary artery smooth muscle cell based assay, a lymphoblastoid cell based assay, or a late-outgrowth endothelial progenitor cell based assay (*see, e.g.,* Drake et al., 2013 Am J Respir Cell Mol Biol, 49(3): 403-409).

Examples of biological assays for nonsense mutation-mediated diseases, including cystic fibrosis, include, but are not limited to a HeLa cell based assay containing fusion protein MS2-UPF, Calu-3 and Calu-6 cell based assays, a DMD cell based assay (*see, e.g.,* Gonzalez-Hilarion et al., 2012 Orphanet Journal of Rare Diseases, 7(58); 1-14).

An example of a biological assay for cystic fibrosis includes, but is not limited to assays utilizing the peripheral blood monocytes, lymphocytes and polymorphonuclear cells of cystic fibrosis patients (*see, e.g.,* Johansson et al., 2014 Cytometry Part A, 85A: 611-620).

An example of a biological assay for xeroderma pigmentosum includes, but is not limited to a XP-C cell based assay (*see, e.g.,* Kuschal et al., 2013 Proc. Natl. Acad. Sci., 110(48): 19483-19488).

Examples of biological assays for late infantile ceroid lipofuscinoses include, but is not limited to INCL, LINCL, and JNCL lymphoblast cell based assays (*see, e.g.,* Miller et al., 2013 Hum. Mol. Gen., 22(13): 2723-2734).

An example of a biological assay for choroideremia includes, but is not limited to a multiplex ligation-dependent probe amplification assay of the CHM gene (*see, e.g.,* Moosajee et al., 2014 Eur. J. Hum. Genet., 22: e1-e4) and the use of INCL cells from patients carrying PPT1 nonsense-mutations (*see, e.g.,* Sarkar et al., 2011 Mol Genet Metab., 104(3): 338-345.).

An example of a biological assay for carnitine palmitoyltransferase 1A deficiency includes, but is not limited to a fibroblast based assay from a CPT1A deficient patient with the homozygous mutation 478 C>T (R160X) (*see, e.g.,* Tan et al., 2011 J. Inherit. Metab. Dis., 34:443-447).

An example of a biological assay for long QT syndrome includes, but is not limited to a transfected HEK293 cell based assay (*see, e.g.,* Yu et al., 2014 Int. J. Mol. Med., 33: 729-735).

An example of a biological assay for propionic acidemia includes, but is not limited to a transcription-translation assay using PCCA cDNA (*see, e.g.,* Sanchez-Alcudia et al., 2012 Hum. Mut., 33(6): 973-980).

Animal model systems can also be used to demonstrate the safety and efficacy of compounds of formula I and II. The compounds of formula I and II can be tested for biological activity using animal models for a disease, condition, or syndrome of interest, as is commonly known to those skilled in the art. These include animals engineered to contain the target RNA element coupled to a functional readout system, such as a transgenic mouse.

Examples of animal models for nonsense mutation mediated diseases include, but are not limited to, the ApcMin mouse model, the Bmp5se mouse model, the Cdh23v-6J mouse model, the C57BL/6J mouse model, the Clcnladr-mto mouse model (*see* Heller et al., 1982 J Neurosci., 2(7):924-33, the B6.Cg-Col4a5tm1Yseg/J mouse model, the CrygdLop12 mouse model, the Crygsrncat mouse model, the C.B6-Enpplb/J mouse model (for ossification of the posterior longitudinal ligament of the spine ("OPLL") *see* Okawa et al., 1998 Nat. Genet., 19(3):271-3), the Foxclch mouse model, the Galc(twi) mouse model, the Gpnmbipd mouse model, the 129S1.B6(C3Fe)-Grhl21Nisw/J mouse model, but are not limited to, the Hps3coa-5J mouse model, the Hps4le mouse model (for Hermansky-Pudlak syndrome type IV, *see* Suzuki et al., 2002 Nature Genet., 30: 321-324), the Il12rb2Ifnm-d mouse model (for inflammatory diseases, *see* Poltorak et al., 2001, J. Immunol.,167(4):2106-1), the C3.B6-Ispdm1Ddg/J mouse model, the Lepob mouse model, the Mitfmi-ce mouse model (for Waardenburg Syndrome type II include, *see* Steingrimsson et al., 1994 Nat. Genet., 8(3): 256-63), the Mitfmi-di mouse model, the C57BL/6-Myo3atm1Mckg/ mouse model, the Npr3lgj-2J mouse model, the op mouse model, the Pax6Coop mouse model, the Pde6brd1 mouse model, the Pldnpa mouse model (*see* Huang, et al., 1999 Nat. Genet., 23(3):329-32), the Polid mouse model, the Prkdcscid mouse model, the Rab23opb mouse model (*see* Eggenschwiler, et al., 2001 Nature, 412(6843):194-8), the Rab23opb2 mouse model, the Smad6b2b390Clo mouse model, the Sobpjc-2J mouse model, the Spnb1ja mouse model, the Spnb4qv mouse model, the Spnb4qv-4J mouse model (for auditory and motor neuropathies, *see* Parkinson, et al., 2001 Nat. Genet., 29(1):61-5), the Sptalsph-ha mouse model, the Tdrd7 mutant mouse model (for cataract and glaucoma, *see* De Angelis et al., 2000 Nat. Genet., 25(4):444-7; Lachke, et al., 2011 Science, 331(6024):1571-6.), the FVB.129X1(B6)-Trfr2tm1Slu/J mouse model, the Zbtb16lu mouse model (for preaxial polydactyly or polydactyly). Described herein are methods of treating, preventing or managing diseases or disorders associated with one or more of the animal models provided herein in a patient, which comprise administering to a patient in need thereof an effective amount of a compound of formula **I** or **II** or a pharmaceutically acceptable salt, hydrate, solvate, clathrate, prodrug, polymorph, stereoisomers, including enantiomers, diastereomers, racemates and mixtures of stereoisomers, thereof.

Examples of animal models for cystic fibrosis include, but are not limited to, G542X-hCFTR mice (*see* Du et al., 2002 J. Mol. Med., 80(9): 595-604; Du et al., 2008 Proc. Natl. Acad. Sci. U.S.A., 105: 2064-2069); cftr(-/-) mice (*see, e.g.,* Freedman et al., 2001, Gastroenterology 121(4):950-7), cftr(tm1HGU/tm1HGU) mice (*see, e.g.,* Bernhardet al., 2001, Exp Lung Res 27(4):349-66), CFTR-deficient mice with defective cAMP-mediated Cl(-) conductance (*see, e.g.,* Stotland et al., 2000, Pediatr Pulmonol 30(5):413-24), and C57BL/6-Cftr(m1UNC)/Cftr(m1UNC) knockout mice (*see, e.g.,* Stotland et al., 2000, Pediatr Pulmonol 30(5):413-24).

Examples of animal models for muscular dystrophy include, but are not limited to, mouse, hamster, cat, dog, and C. *elegans.* Examples of mouse models for muscular dystrophy include, but are not limited to, mdx mouse (*see, e.g*., Welch et al. 2007, 2007 Nature, 447(7140):87-91; Kayali et al., 2012 Hum. Mol. Gen., 21(18):4007-4020 and Li et al., 2014 MedChemComm, 5(8):1075-1091; Nakamura et al., 2001 Neuromuscul. Disord., 11(3):251-9), the dy-/- mouse (*see, e.g.,* Connolly et al., 2002, J Neuroimmunol 127(1-2):80-7), a muscular dystrophy with myositis (mdm) mouse mutation (*see, e.g.,* Garvey et al., 2002, Genomics 79(2):146-9), the utrophin-dystrophin knockout (dko) mouse (*see, e.g.,* Nakamura et al., 2001, Neuromuscul Disord 11(3):251-9), the dy/dy mouse (*see, e.g.,* Dubowitz et al., 2000, Neuromuscul Disord 10(4-5):292-8), the mdx(Cv3) mouse model (*see, e.g.,* Pillers et al., 1999, Laryngoscope 109(8):1310-2), the DMD (mdx-4Cv) mouse model, and the myotonic ADR-MDX mutant mice (*see, e.g.,* Kramer et al., 1998, Neuromuscul Disord 8(8):542-50).

Examples of animal models for familial hypercholesterolemia include, but are not limited to, mice lacking functional LDL receptor genes (*see, e.g.,* Aji et al., 1997, Circulation 95(2):430-7), Yoshida rats (*see, e.g.,* Fantappie et al., 1992, Life Sci 50(24):1913-24), the JCR:LA-cp rat (*see, e.g.,* Richardson et al., 1998, Atherosclerosis 138(1):135-46), swine *(see, e.g.,* Hasler-Rapacz et al., 1998, Am J Med Genet 76(5):379-86), and the Watanabe heritable hyperlipidaemic rabbit (*see, e.g.,* Tsutsumi et al., 2000, Arzneimittelforschung 50(2):118-21; Harsch et al., 1998, Br J Pharmacol 124(2):227-82; and Tanaka et al., 1995, Atherosclerosis 114(1):73-82).

An example of an animal model for human cancer in general includes, but is not limited to, Familial adenomatous polyposi mouse model (*see, e.g.,* Moser et al., 1990 Science., 247(4940):322-4), spontaneously occurring tumors of companion animals (*see, e.g.,* Vail & MacEwen, 2000, Cancer Invest 18(8):781-92). Examples of animal models for lung cancer include, but are not limited to, lung cancer animal models described by Zhang & Roth (1994, In Vivo 8(5):755-69) and a transgenic mouse model with disrupted p53 function (*see, e.g.,* Morris et al., 1998, J La State Med Soc 150(4):179-85). An example of an animal model for breast cancer includes, but is not limited to, a transgenic mouse that overexpresses cyclin D1 (s*ee, e.g.,* Hosokawa et al., 2001, Transgenic Res 10(5):471-78). An example of an animal model for colon cancer includes, but is not limited to, a TCRbeta and p53 double knockout mouse (*see, e.g.,* Kado et al., 2001, Cancer Res 61(6):2395-98). Examples of animal models for pancreatic cancer include, but are not limited to, a metastatic model of Panc02 murine pancreatic adenocarcinoma (*see, e.g.,* Wang et al., 2001, Int J Pancreatol 29(1):37-46) and nu-nu mice generated in subcutaneous pancreatic tumours (*see, e.g.,* Ghaneh et al., 2001, Gene Ther 8(3):199-208). Examples of animal models for non-Hodgkin's lymphoma include, but are not limited to, a severe combined immunodeficiency ("SCID") mouse (*see, e.g.,* Bryant et al., 2000, Lab Invest 80(4):553-73) and an IgHmu-HOX11 transgenic mouse (*see, e.g.,* Hough et al., 1998, Proc Natl Acad Sci USA 95(23):13853-58). An example of an animal model for esophageal cancer includes, but is not limited to, a mouse transgenic for the human papillomavirus type 16 E7 oncogene (*see, e.g.,* Herber et al., 1996, J Virol 70(3):1873-81). Examples of animal models for colorectal carcinomas include, but are not limited to, Apc mouse models (*see, e.g.,* Fodde & Smits, 2001, Trends Mol Med 7(8):369-73 and Kuraguchi et al., 2000, Oncogene 19(50):5755-63). An example of an animal model for neurofibromatosis includes, but is not limited to, mutant NF1 mice (*see, e.g.,* Cichowski et al., 1996, Semin Cancer Biol 7(5):291-8). Examples of animal models for retinoblastoma include, but are not limited to, transgenic mice that expression the simian virus 40 T antigen in the retina (*see, e.g.,* Howes et al., 1994, Invest Ophthalmol Vis Sci 35(2):342-51 and Windle et al, 1990, Nature 343(6259):665-69) and inbred rats (*see, e.g.,* Nishida et al., 1981, Curr Eye Res 1(1):53-55 and Kobayashi et al., 1982, Acta Neuropathol (Berl) 57(2-3):203-08). Examples of animal models for Wilm's tumor include, but are not limited to, a WT1 knockout mice (*see, e.g.,* Scharnhorst et al., 1997, Cell Growth Differ 8(2):133-43), a rat subline with a high incidence of neuphroblastoma (*see, e.g.,* Mesfin & Breech, 1996, Lab Anim Sci 46(3):321-26), and a Wistar/Furth rat with Wilms' tumor (*see, e.g.,* Murphy et al., 1987, Anticancer Res 7(4B):717-19).

An example of an animal model for Usher syndrome includes, but is not limited to the C57BL/6J mouse model (*see, e.g.,* Goldmann, et al., 2011 Human Gene Therapy, 22:537-547 and Goldmann, et al., 2012 EMBO Mol. Med., 4(11): 1186-1199).

An example of an animal model for pseudoxanthoma elasticum includes, but is not limited to a zebrafish mRNA rescue assay (*see, e.g.,* Zhou et al., 2013 J. Invest. Derm., 133(12): 2672-2677).

An example of an animal model for aniridia includes, but is not limited to the pax6 mouse model (*see, e.g.,* Gregory-Evans et al., 2014 J. Clin. Invest., 124(1); 111-116).

An example of an animal model for methylmalonic aciduria includes, but is not limited to the transgenic mouse model carrying the human R403stop mutation on the methylmalonyl-CoA mutase (MCM) locus (*see, e.g.,* Buck et al., 2012 Biochem. Biophys. Res. Commun 427(4): 753-757.

### 4.3 PHARMACEUTICAL COMPOSITIONS

Pharmaceutical compositions and single unit dosage forms comprising a a compound of formula II provided herein, or a pharmaceutically acceptable salt, hydrate, solvate, clathrate, polymorph, stereoisomers, including enantiomers, diastereomers, racemates and mixtures of stereoisomers, thereof, are also provided herein. Individual dosage forms provided herein may be suitable for oral, mucosal (including sublingual, buccal, rectal, nasal, or vaginal), parenteral (including subcutaneous, intramuscular, bolus injection, intraarterial, or intravenous), transdermal, or topical (including ocular) administration.

Single unit dosage forms provided herein are suitable for oral, mucosal (*e.g*., nasal, sublingual, vaginal, buccal, or rectal), parenteral (*e.g*., subcutaneous, intravenous, bolus injection, intramuscular, or intraarterial), transdermal or topical (including ocular) administration administration to a patient.

The composition, shape, and type of dosage forms provided herein will typically vary depending on their use. These and other ways in which specific dosage forms provided herein may vary from one another will be readily apparent to those skilled in the art. *See, e.g.,* Remington's Pharmaceutical Sciences, 18th ed., Mack Publishing, Easton PA (1995).

Typical pharmaceutical compositions and dosage forms comprise one or more carriers, excipients or diluents. Suitable excipients are well known to those skilled in the art of pharmacy, and non-limiting examples of suitable excipients are provided herein. Whether a particular excipient is suitable for incorporation into a pharmaceutical composition or dosage form depends on a variety of factors well known in the art including, but not limited to, the way in which the dosage form will be administered to a patient. For example, oral dosage forms such as tablets may contain excipients not suited for use in parenteral dosage forms. The suitability of a particular excipient may also depend on the specific active ingredients in the dosage form.

### 4.4 COMPOUNDS FOR USE IN METHODS OF TREATING

Described herein are methods of treating, preventing or managing diseases or disorders ameliorated by the suppression of premature translation termination in a patient which comprise administering to a patient in need thereof an effective amount of a compound of formula **I** or **II** or a pharmaceutically acceptable salt, hydrate, solvate, clathrate, prodrug, polymorph, stereoisomers, including enantiomers, diastereomers, racemates and mixtures of stereoisomers, thereof.

In one embodiment, provided herein are the compounds provided herein for use in methods for the treatment, prevention or management of any disease that is associated with a gene exhibiting premature translation termination, wherein the disease is due, in part, to the lack of expression of the gene resulting from a premature stop codon. Specific examples of genes which may exhibit premature translation termination and diseases associated with premature translation termination are found in U.S. Patent No. 7,291,461, titled: "Methods For Identifying Small Molecules That Modulate," issued November 6, 2007.

In one embodiment, the disease or disorder associated with a premature stop codon is a cancer, a muscular dystrophy, a lysosomal storage disease, an autoimmune disease, a blood disease, a kidney disease, a collagen disease, diabetes, a central nervous system disease, an ocular disease, a neurodegenerative disease, a proliferative disease, a cardiovascular disease, a pulmonary disease, an inflammatory disease or a central nervous system disease.

In one embodiment, the lysosomal storage disease is selected from tuberous sclerosis, mucopolysaccharaidosis type I (Hurler's Syndrome), mucopolysaccharidosis type III A, mucopolysaccharidosis type VI, mucopolysaccharidosis type VII, metachromatic leukodystrophy, Niemann Pick's disease C, or Sandhoff disease; the autoimmune disease is selected from rheumatoid arthritis or graft versus host disease; the blood disease is selected from hemophilia A, hemophilia B, Von Willebrand disease, or ataxia-telangiectasiab-thalassemia; the kidney disease is selected from kidney stones, polycystic kidney disease, or Dent Disease; the collagen disease is selected from osteogenesis imperfecta, Marfan Syndrome, or cirrhosis; the inflammatory disease is arthritis; the central nervous system disease is selected from multiple sclerosis, infantile neuronal ceroid lipofuscinoses, late infantile neuronal ceroid lipofuscinosis, ataxia telangiectasia, Usher Syndrome, Alzheimer's disease, Tay Sachs disease, Parkinson's disease, Krabbe's disease, congenital hydrocephalus, or Menkes Syndrome; the pulmonary disease is selected from cystic fibrosis, long QT syndrome, heritable pulmonary arterial hypertension, or heart disease; the muscular dystrophy is Duchenne muscular dystrophy; the ocular disease is selected from Waardenburg Syndrome type II, iris pigment dispersion, aniridia, choroideremia, renal-coloboma syndrome, Lebers congenital amaurosis, retinitis pigmentosa, Bardet-Biedl syndrome, glaucoma, foveal hypoplasia, cataracts, central auditory processing difficulties, chorioretinal degeneration, congenital lens opacities, elevated intraocular pressure, exudative vascular retinopathy, iris hypoplasia, keratopathy (corneal degeneration), optic nerve hypoplasia, retinal detachment, secondary strabismus, gyrate atrophy, or tunica vasculosa lentis; the cancer is of the head and neck, eye, skin, mouth, throat, esophagus, chest, bone, lung, colon, sigmoid, rectum, stomach, prostate, breast, ovaries, kidney, liver, pancreas, brain, intestine, heart, adrenals; or, the cancer is a solid tumor selected from a sarcoma, carcinoma, fibrosarcoma, myxosarcoma, liposarcoma, chondrosarcoma, osteogenic sarcoma, chordoma, angiosarcoma, endotheliosarcoma, lymphangiosarcoma, lymphangioendotheliosarcoma, synovioma, mesothelioma, Ewing's tumor, leiomyosarcoma, rhabdomyosarcoma, squamous cell carcinoma, basal cell carcinoma, adenocarcinoma, sweat gland carcinoma, sebaceous gland carcinoma, papillary carcinoma, papillary adenocarcinomas, cystadenocarcinoma, medullary carcinoma, bronchogenic carcinoma, renal cell carcinoma, hepatoma, bile duct carcinoma, choriocarcinoma, seminoma, embryonal carcinoma, Wilms' tumor, cervical cancer, testicular tumor, lung carcinoma, small cell lung carcinoma, bladder carcinoma, epithelial carcinoma, glioma, astrocytoma, medulloblastoma, craniopharyngioma, ependymoma, Kaposi's sarcoma, pinealoma, hemangioblastoma, acoustic neuroma, oligodendroglioma, menangioma, melanoma, neuroblastoma or retinoblastoma, or a blood-born tumor selected from acute lymphoblastic leukemia, acute lymphoblastic B-cell leukemia, acute lymphoblastic T-cell leukemia, acute myeloblastic leukemia, acute promyelocytic leukemia, acute monoblastic leukemia, acute erythroleukemic leukemia, acute megakaryoblastic leukemia, acute myelomonocytic leukemia, acute nonlymphocyctic leukemia, acute undifferentiated leukemia, chronic myelocytic leukemia, chronic lymphocytic leukemia, rhabdoid tumor, hairy cell leukemia, or multiple myeloma; the metabolic disorder is selected from methylmalonic aciduria, type II diabetes and obesity, acute intermittent porphyria, pyruvate kinase deficiency, propionic acidemia, carnitine palmitoyltransferase 1A deficiency, or adrenoleukodystrophy; the skin disease is selected from pseudoxanthoma elasticum, epidermolysis bullosa, Herlitz epidermolysis bullosa, xeroderma pigmentosum, or ectodermal dysplasia/skin fragility syndrome; the neuropathic disease is selected from auditory and motor neuropathies; the neuromuscular disorder is selected from myotonia and spinal muscular atrophy; in one embodiment, the disease is selected from preaxial polydactyly or polydactyly, hemochromatosis, Hermansky-Pudlak syndrome (type III and IV), ossification of the posterior longitudinal ligament of the spine, multiple endocrine neoplasia (type 1, 2 and 3), amyloidosis, congenital adrenal hypoplasia, adenomatous poliposis coli, Von Hippel Landau Disease, collagen VII, Alagille Syndrome, Townes-Brocks Syndrome, Coffin-Lowry Syndrome, Charcot-Maria-Tooth Disease, myotubular myopathy, X-linked myotubular myopathy, X-linked chondrodysplasia, X-linked agammaglobulinemia, familial adenomatous poliposis, pyruvate dehydrogenase deficiency, phenylketonuria, neurofibromatosis 1, neurofibromatosis 2, Rett Syndrome, Leri-Weill dyschondrosteosis, rickets, hypophosphatemic rickets, atherosclerosis, sensorineural deafness, dystonia, Cowden Disease, Wilson Disease, Treacher-Collins Syndrome, giantism, dwarfism, hypothyroidism, hyperthyroidism, aging, ataxia-telangiectasia, or familial hypercholesterolemia.

In one embodiment, the nmMPS I disease results from certain nonsense mutations on one or both alleles of the IDUA gene selected from Q60X, Y64X, Q70X, Y167X, Q310X, Q320X, Q400X, W402X, G409X,Y581X, R619X, R621X, R626X, R628X and the like.

In one embodiment, the cancer is associated with a tumor suppressor gene encoding a premature stop codon, and wherein the tumor suppressor gene is selected from APC, ATM, BRAC1, BRAC2, MSH1, pTEN, Rb, CDKN2, NF1, NF2, WT1 or p53.

In one embodiment, the disease or disorder associated with a premature stop codon is cystic fibrosis or Duchenne muscular dystrophy.

In a specific embodiment, the methods, compositions, doses, unit dosage forms and dosing regimens provided herein are useful for the treatment, prevention or management of a disease associated with a nonsense mutation in a gene in an embryo or fetus who has or is predisposed or susceptible to a disease associated with a nonsense mutation in a gene, such as those described herein. In accordance with this embodiment, a pregnant female is administered an effective amount a compound of formula **II** provided herein or a pharmaceutically acceptable salt, hydrate, solvate, clathrate, polymorph, stereoisomers, including enantiomers, diastereomers, racemates and mixtures of stereoisomers, thereof which passes through the placenta to the embryo or fetus. In a particular embodiment, an effective amount of a compound of formula **II** provided herein or a pharmaceutically acceptable salt, hydrate, solvate, clathrate, polymorph, stereoisomers, including enantiomers, diastereomers, racemates and mixtures of stereoisomers, thereof is administered orally to the pregnant female.

In another embodiment, the cancer is associated with tumor suppressor genes (*see, e.g.,* Garinis et al. 2002, Hum Gen 111:115-117; Meyers et al. 1998, Proc. Natl. Acad. Sci. USA, 95: 15587-91; Kung et al. 2000, Nature Medicine 6(12): 1335-40. Such tumor suppressor genes include, but are not limited to, APC, ATM, BRAC1, BRAC2, MSH1, pTEN, Rb, CDKN2, NF1, NF2, WT1, and p53.

In a particular embodiment, the tumor suppressor gene is the p53 gene. Nonsense mutations have been identified in the p53 gene and have been implicated in cancer. Several nonsense mutations in the p53 gene have been identified (*see, e.g.,* Masuda et al., 2000, Tokai J Exp Clin Med. 25(2):69-77; Oh et al., 2000, Mol Cells 10(3):275-80; Li et al., 2000, Lab Invest. 80(4):493-99; Yang et al., 1999, Zhonghua Zhong Liu Za Zhi 21(2):114-18; Finkelstein et al., 1998, Mol Diagn. 3(1):37-41; Kajiyama et al., 1998, Dis Esophagus. 11(4):279-83; Kawamura et al., 1999, Leuk Res. 23(2):115-26; Radig et al., 1998, Hum Pathol. 29(11):1310-16; Schuyer et al., 1998, Int J Cancer 76(3):299-303; Wang-Gohrke et al., 1998, Oncol Rep. 5(1):65-68; Fulop et al., 1998, J Reprod Med. 43(2):119-27; Ninomiya et al., 1997, J Dermatol Sci. 14(3):173-78; Hsieh et al., 1996, Cancer Lett. 100(1-2):107-13; Rall et al., 1996, Pancreas. 12(1):10-17; Fukutomi et al., 1995, Nippon Rinsho. 53(11):2764-68; Frebourg et al., 1995, Am J Hum Genet. 56(3):608-15; Dove et al., 1995, Cancer Surv. 25:335-55; Adamson et al., 1995, Br J Haematol. 89(1):61-66; Grayson et al., 1994, Am J Pediatr Hematol Oncol. 16(4):341-47; Lepelley et al., 1994, Leukemia. 8(8):1342-49; McIntyre et al., 1994, J Clin Oncol. 12(5):925-30; Horio et al., 1994, Oncogene. 9(4):1231-35; Nakamura et al., 1992, Jpn J Cancer Res. 83(12):1293-98; Davidoff et al., 1992, Oncogene. 7(1):127-33; and Ishioka et al., 1991, Biochem Biophys Res Commun. 177(3):901-06).

In one embodiment, provided herein are the compounds provided herein for use in a method of treating, preventing or reducing cough, comprising administering an effective amount of a compound of formula **II** provided herein or a pharmaceutically acceptable salt, hydrate, solvate, clathrate, polymorph, stereoisomers, including enantiomers, diastereomers, racemates and mixtures of stereoisomers, thereof to a patient in need thereof. In a particular embodiment, the patient has cystic fibrosis. In another embodiment, the cough is chronic cough. In another embodiment, a compound of formula **II** provided herein or a pharmaceutically acceptable salt, hydrate, solvate, clathrate, polymorph, stereoisomers, including enantiomers, diastereomers, racemates and mixtures of stereoisomers, thereof is administered as a dosage form provided herein or according to a dosing regimen provided herein.

In one embodiment, provided herein are the compounds provided herein for use in a method of increasing dystrophin expression in muscle, comprising administering an effective amount of a compound of formula **II** provided herein or a pharmaceutically acceptable salt, hydrate, solvate, clathrate, polymorph, stereoisomers, including enantiomers, diastereomers, racemates and mixtures of stereoisomers, thereof to a patient in need thereof. In a particular embodiment, provided herein are the compounds provided herein for use in methods of increasing dystrophin expression in muscle cells, comprising contacting the muscle cells with an effective amount of a compound of formula **II** provided herein or a pharmaceutically acceptable salt, hydrate, solvate, clathrate, polymorph, stereoisomers, including enantiomers, diastereomers, racemates and mixtures of stereoisomers, thereof. In one embodiment, the muscle cells are contacted *in vitro.* In a specific embodiment, the patient has Duchenne muscular dystrophy. In another embodiment, a compound of formula **II** provided herein or a pharmaceutically acceptable salt, hydrate, solvate, clathrate, polymorph, stereoisomers, including enantiomers, diastereomers, racemates and mixtures of stereoisomers, thereof is administered as a dosage form provided herein or according to a dosing regimen provided herein.

### 4.5 DOSES AND DOSING REGIMENS

Without being limited by theory, provided herein are, in part, specific doses and dosing regimens for a compound of formula **II** provided herein or a pharmaceutically acceptable salt, hydrate, solvate, clathrate, polymorph, stereoisomers, including enantiomers, diastereomers, racemates and mixtures of stereoisomers, thereof that optimize the suppression of premature translation termination

The inventions provided herein encompass the treatment, prevention, and management of diseases treatable or preventable by the suppression of premature translation termination or symptoms thereof while reducing or avoiding adverse or unwanted effects, *e.g.,* toxicities or side effects. The preferred route of administration for the doses and dosing regimens described herein is oral (*i.e.,* ingestion of a solution, a colloid solution or a solution with additional active agent, above the saturating concentration of active agent).

The doses and dosing regimens described herein are thought to be useful due to their ability to achieve and maintain a desirable plasma concentration of the active agent. Without being limited by theory, it is thought that achieving and maintaining a relatively constant plasma concentration of active agent (such as those described in Section 4.1) over, for example, a 24 hour period or longer, provides a beneficial therapeutic effect to the patient. The doses and dosing regimens described herein are useful for achieving and maintaining such therapeutic plasma concentrations of active agent.

In one embodiment, provided herein are the compounds provided herein for use in a method of administering a compound of formula **II** provided herein or a pharmaceutically acceptable salt, hydrate, solvate, clathrate, polymorph, stereoisomers, including enantiomers, diastereomers, racemates and mixtures of stereoisomers, thereof wherein the active agent is administered to a patient in need thereof once in a 12 or 24 hour period.

In another embodiment, provided herein are the compounds provided herein for use in a method of administering a compound of formula **II** provided herein or a pharmaceutically acceptable salt, hydrate, solvate, clathrate, polymorph, stereoisomers, including enantiomers, diastereomers, racemates and mixtures of stereoisomers, thereof wherein the active agent is administered to a patient in need thereof one or two times in a 12 or 24 hour period, wherein each administration is preferably separated by about 4-14 hours, in one embodiment 12 hours. In these embodiments, the active agent can be administered, for example, at meal time, such as breakfast, lunch and supper.

In another embodiment, provided herein are the compounds provided herein for use in a method of administering a compound of formula **II** provided herein or a pharmaceutically acceptable salt, hydrate, solvate, clathrate, polymorph, stereoisomers, including enantiomers, diastereomers, racemates and mixtures of stereoisomers, thereof wherein the active agent is administered to a patient in need thereof three times in a 12 or 24 hour period, wherein each administration is preferably separated by about 4-14 hours. In a particular embodiment, the active agent is administered once in the morning, once in the afternoon and once in the evening. Preferred intervals between doses include 4, 5, 6, 7, 8, 9, 10, 11, 12, 13 and 14 hours.

In one embodiment, the dose of active agent is escalated throughout a 24 hour period. In another embodiment, the second dose administered is escalated (e.g., doubled). In another embodiment, the first and second dose administered are kept constant and the third dose administered is escalated (e.g., doubled). In a particular embodiment, the three doses in a 24 hour period are administered according to the formula: 1X, 1X, 2X, where X is a particular initial dose (*e.g.,* 4 mg/kg, 5 mg/kg, 7mg/kg, 10 mg/kg, 20 mg/kg, 25 mg/kg, 30 mg/kg, 35 mg/kg, 40 mg/kg or 50 mg/kg). In another embodiment, the active agent is administered within (*i.e.,* before or after) about 10, 15, 30, 45 or 60 minutes of the patient having food. In one embodiment, an effective amount of the active agent is sprinkled on or mixed in food. In another embodiment, the active agent is administered without food.

A particularly preferred dosing regimen is that where a patient is administered the active agent within 30 minutes after a meal at approximately 6, 6, and 12 hour intervals (e.g., at ~7:00 AM after breakfast, ~1:00 PM after lunch, and at ~7:00 PM after supper).

In yet another embodiment, provided herein is the administration of a compound of formula **II** provided herein or a pharmaceutically acceptable salt, hydrate, solvate, clathrate, polymorph, stereoisomers, including enantiomers, diastereomers, racemates and mixtures of stereoisomers, thereof in single or divided (*e.g*., three times in a 24 hour period) doses between 0.1 mg/kg and 1500 mg/kg, 0.1 mg/kg and 1200 mg/kg, 0.1 mg/kg and 1000 mg/kg, 0.1 mg/kg and 750 mg/kg, 0.1 mg/kg and 500 mg/kg, 1 mg/kg and 250 mg/kg, 1 mg/kg and 150 mg/kg, 1 mg/kg and 100 mg/kg, 1 mg/kg and 50 mg/kg, 1 mg/kg and 25 mg/kg, 1 mg/kg and 10 mg/kg or 2 mg/kg and 10 mg/kg to a patent in need thereof.

In a particular embodiment, a compound of formula **II** provided herein or a pharmaceutically acceptable salt, hydrate, solvate, clathrate, polymorph, stereoisomers, including enantiomers, diastereomers, racemates and mixtures of stereoisomers, thereof is administered in a dose of about 2-6 mg/kg, about 5-9 mg/kg, about 6-10 mg/kg, about 8-12 mg/kg, about 12-16 mg/kg or about 18-22 mg/kg.

In a particular embodiment, a compound of formula **II** provided herein or a pharmaceutically acceptable salt, hydrate, solvate, clathrate, polymorph, stereoisomers, including enantiomers, diastereomers, racemates and mixtures of stereoisomers, thereof is administered in a dose of about 4 mg/kg, about 7 mg/kg, about 8 mg/kg, about 10 mg/kg, about 14 mg/kg, about 20 mg/kg, about 25 mg/kg, about 30 mg/kg, about 35 mg/kg, about 40 mg/kg or about 50 mg/kg. In another embodiment, any dose of a compound of formula **II** provided herein or a pharmaceutically acceptable salt, hydrate, solvate, clathrate, polymorph, stereoisomers, including enantiomers, diastereomers, racemates and mixtures of stereoisomers, thereof described in the preceding embodiment is administered three times in a 24 hour period.

In another embodiment, provided herein is a continuous therapy wherein a compound of formula **II** provided herein or a pharmaceutically acceptable salt, hydrate, solvate, clathrate, polymorph, stereoisomers, including enantiomers, diastereomers, racemates and mixtures of stereoisomers, thereof is administered daily to a patient in need thereof for a certain period of time (*e.g.,* 5, 7, 10, 14, 20, 24, 28, 60 or 120 days or more). In one embodiment, the active agent is continuously administered three times per 24 hour period. In another embodiment, the active agent is administered continuously daily, weekly, monthly or yearly.

In a specific embodiment, the active agent is continuously administered three times per 24 hour period at doses of about 4 mg/kg, about 4 mg/kg and about 8 mg/kg for days, weeks, months or years. In a specific embodiment, the active agent is continuously administered three times per 24 hour period at doses of about 7 mg/kg, about 7 mg/kg and about 14 mg/kg for days, weeks, months or years. In a specific embodiment, the active agent is continuously administered three times per 24 hour period at doses of about 10 mg/kg, about 10 mg/kg and about 20 mg/kg for days, weeks, months or years. In a specific embodiment, the active agent is continuously administered three times per 24 hour period at doses of about 30 mg/kg, about 30 mg/kg and about 60 mg/kg for days, weeks, months or years. In a specific embodiment, the active agent is continuously administered three times per 24 hour period at doses of about 10 mg/kg, about 10 mg/kg and about 20 mg/kg for days, weeks, months or years. In a specific embodiment, the active agent is continuously administered three times per 24 hour period at doses of about 40 mg/kg, about 40 mg/kg and about 80 mg/kg for days, weeks, months or years. In each 24 hour period that the active agent is administered, it is preferably administered three times at approximately 6, 6, and 12 hour intervals (e.g., at ~7:00 AM after breakfast, ~1:00 PM after lunch, and at ~7:00 PM after supper). Continuous therapy is preferably used for the treatment, prevention or management of Cystic Fibrosis and Duchenne Muscular Dystrophy.

Treatment periods for a course of therapy can span one week, two weeks, three weeks, four weeks, five weeks, six weeks, seven weeks, eight weeks, nine weeks, ten weeks, eleven weeks, twelve weeks, thirteen weeks, fourteen weeks, four months, five months, six months, seven months, eight months, nine months, ten months, eleven months, one year, two years, three years, four years, five years or longer. The treatment periods can be interrupted by periods of rest which can span a day, one week, two weeks, three weeks, four weeks, five weeks, six weeks, seven weeks, eight weeks, nine weeks, ten weeks, eleven weeks, twelve weeks, thirteen weeks, fourteen weeks, four months, five months, six months, seven months, eight months, nine months, ten months, eleven months, one year, two years, three years, four years, five years or longer. Such determinations can be made by one skilled in the art (*e.g.,* a physician).

In certain embodiments, a compound of formula **II** provided herein or a pharmaceutically acceptable salt, hydrate, solvate, clathrate, polymorph, stereoisomers, including enantiomers, diastereomers, racemates and mixtures of stereoisomers, thereof is administered according to the doses and dosing schedules described herein in combination with a second active agent (*e.g*., simultaneously or sequentially). In particular embodiments, a compound of formula **II** provided herein or a pharmaceutically acceptable salt, hydrate, solvate, clathrate, polymorph, stereoisomers, including enantiomers, diastereomers, racemates and mixtures of stereoisomers, thereof is administered according to the doses and dosing schedules described herein in combination with an aminoglycoside, a corticosteroid, a pancreatic enzyme, an antibiotic, insulin, a hypoglycemic agent, an omega-3 fatty acid, a chemotherapeutic agent, or an enzyme replacement therapy. The administration of the second active agent can be topical, enteral (*e.g.,* oral, duodenal, rectal), parenteral (*e.g.,* intravenous, intraarterial, intramuscular, subcutaneous, intradermal or interaperitoneal) or intrathecal. In certain embodiments, a compound of formula **II** provided herein or a pharmaceutically acceptable salt, hydrate, solvate, clathrate, polymorph, stereoisomers, including enantiomers, diastereomers, racemates and mixtures of stereoisomers, thereof is administered according to the doses and dosing schedules described herein in combination with radiation therapy.

It will be understood that the amounts of active agent administered to a patient in need thereof are or can be calculated based upon the actual weight of the patient in question or the average weight of the patient population.

### 4.6 PLASMA CONCENTRATIONS

Also described herein is a method of maintaining a therapeutically beneficial plasma concentration of a compound of formula **I** or **II** or a pharmaceutically acceptable salt, hydrate, solvate, clathrate, prodrug, polymorph, stereoisomers, including enantiomers, diastereomers, racemates and mixtures of stereoisomers, thereof in an amount greater than: about 0.1 µg/mL, about 0.5 µg/mL, about 2 µg/mL, about 5 µg/mL, about 10 µg/mL to about 20 µg/mL in a patient for at least about 2, 2.5, 3, 3.5, 4, 4.5, 5, 6, 8, 12 or 24 hours or longer, comprising administering an effective amount of a compound of formula **I** or **II** or a pharmaceutically acceptable salt, hydrate, solvate, clathrate, prodrug, polymorph, stereoisomers, including enantiomers, diastereomers, racemates and mixtures of stereoisomers, thereof to a patient in need thereof. In a particular example, the administration is oral or topical. Levels of a compound of formula **I** or **II** or a pharmaceutically acceptable salt, hydrate, solvate, clathrate, prodrug, polymorph, stereoisomers, including enantiomers, diastereomers, racemates and mixtures of stereoisomers, thereof in plasma can be measured, for example, by high performance liquid chromatography (HPLC).

Also described herein is a method of maintaining a plasma concentration of a compound of formula **I** or **II** or a pharmaceutically acceptable salt, hydrate, solvate, clathrate, prodrug, polymorph, stereoisomers, including enantiomers, diastereomers, racemates and mixtures of stereoisomers, thereof of about 0.1 µg/mL to about 500 µg/mL, about 2 µg/mL to about 10 µg/mL, or about 2 µg/mL to about 20 µg/mL in a patient for at least about 2, 2.5, 3, 3.5, 4, 4.5, 5, 6, 8, 12 or 24 hours or longer, comprising administering an effective amount of a compound of formula **I** or **II** or a pharmaceutically acceptable salt, hydrate, solvate, clathrate, prodrug, polymorph, stereoisomers, including enantiomers, diastereomers, racemates and mixtures of stereoisomers, thereof one, two or three times per day at the same or escalating doses (*e.g.,* 1X, 1X, 2X as described herein). In a particular example, the administration is oral.

In a particular example, a patient's plasma level of a compound of formula **I** or **II** or a pharmaceutically acceptable salt, hydrate, solvate, clathrate, prodrug, polymorph, stereoisomers, including enantiomers, diastereomers, racemates and mixtures of stereoisomers, thereof is maintained above about 2 µg/mL for at least about 2, 2.5, 3, 3.5, 4, 4.5, 5, 6, 8, 12 or 24 hours or longer by administration of the active agent one, two or three times per day to a patient in need thereof. In another example, a patient's plasma level of a compound of formula **I** or **II** or a pharmaceutically acceptable salt, hydrate, solvate, clathrate, prodrug, polymorph, stereoisomers, including enantiomers, diastereomers, racemates and mixtures of stereoisomers, thereof is maintained between about 2 µg/mL to about 10 µg/mL for at least about 2, 2.5, 3, 3.5, 4, 4.5, 5, 6, 8, 12 or 24 hours or longer hours by administration of the active agent one, two or three times per day to a patient in need thereof. In a particular example, a patient's plasma level of a compound of formula **I** or **II** or a pharmaceutically acceptable salt, hydrate, solvate, clathrate, prodrug, polymorph, stereoisomers, including enantiomers, diastereomers, racemates and mixtures of stereoisomers, thereof is maintained above about 10 µg/mL for at least about 2, 2.5, 3, 3.5, 4, 4.5, 5, 6, 8, 12 or 24 hours or longer by administration of the active agent one, two or three times per day to a patient in need thereof. In a particular example, the administration is oral.

In a particular example, described herein are methods for achieving a Cₘₐₓ of 1 µg/mL to 1000 µg/mL, 1 µg/mL to 750 µg/mL, 1 µg/mL to 500 µg/mL, 1 µg/mL to 400 µg/mL, 1 µg/mL to 300 µg/mL, 1 µg/mL to 250 µg/mL, 1 µg/mL to 200 µg/mL, 1 µg/mL to 150 µg/mL, 1 µg/mL to 100 µg/mL, 1 µg/mL to 50 µg/mL, 1 µg/mL to 40 µg/mL, 1 µg/mL to 30 µg/mL, 1 µg/mL to 20 µg/mL, 1 µg/mL to 10 µg/mL, or 10 µg/mL to 30 µg/mL of a compound of formula **I** or **II** or a pharmaceutically acceptable salt, hydrate, solvate, clathrate, prodrug, polymorph, stereoisomers, including enantiomers, diastereomers, racemates and mixtures of stereoisomers, thereof is in a patient comprising administering an effective amount of a compound of formula **I** or **II** or a pharmaceutically acceptable salt, hydrate, solvate, clathrate, prodrug, polymorph, stereoisomers, including enantiomers, diastereomers, racemates and mixtures of stereoisomers, thereof.

In a particular example, described herein are methods for achieving a AUC₀₋₂₄ of 50 µg·hour/mL to 1000 µg·hour/mL, 50 µg·hour/mL to 750 µg·hour/mL, 50 µg·hour/mL to 500 µg·hour/mL, 50 µg·hour/mL to 400 µg·hour/mL, 50 µg·hour/mL to 300 µg·hour/mL, 50 µg·hour/mL to 250 µg·hour/mL, 50 µg·hour/mL to 200 µg·hour/mL, 50 µg·hour/mL to 150 µg·hour/mL, or 50 µg·hour/mL to 100 µg·hour/mL of a compound of formula **I** or **II** or a pharmaceutically acceptable salt, hydrate, solvate, clathrate, prodrug, polymorph, stereoisomers, including enantiomers, diastereomers, racemates and mixtures of stereoisomers, thereof is in a patient comprising administering an effective amount of a compound of formula **I** or **II** or a pharmaceutically acceptable salt, hydrate, solvate, clathrate, prodrug, polymorph, stereoisomers, including enantiomers, diastereomers, racemates and mixtures of stereoisomers, thereof one, two or three times per day to a patient in need thereof.

### 4.7 SYNTHESIS AND PREPARATION

The compounds provided herein can be obtained via standard, well-known synthetic methodology, *see, e.g.,* March, J. Advanced Organic Chemistry; Reactions Mechanisms, and Structure, 4th ed., 1992. Starting materials useful for preparing the compounds provided herein and intermediates therefor, are commercially available or can be prepared from commercially available materials using known synthetic methods and reagents. Compounds of formula **I** and **II** can be synthesized using the synthesis depicted in Scheme A and Scheme B, *infra.* Examples of the synthesis of certain hydrogen isotope-enriched 1,2,4-oxadiazole benzoic acid compounds provided herein are provided in Section 5, *infra.*

Particular methods for synthesizing non-isotopically enriched compounds of formula **I** and formula **II** are disclosed in WO 2004/091502, published on October 28, 2004, and WO 2008/030570, published on March 13, 2008

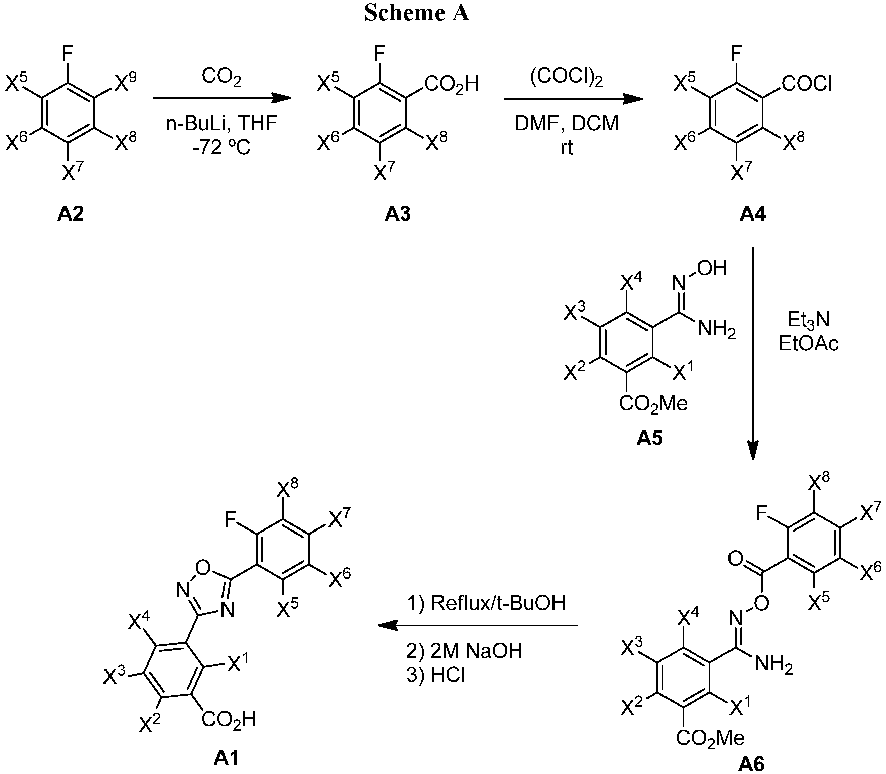

As depicted in Scheme A, the hydrogen isotope-enriched 1,2,4-oxadiazole benzoic acid compounds of formula **I** and **II** have X¹ X², X³, X⁴ as H, wherein X⁵, X⁶, X⁷, and X⁸ is D.

The fluorobenzene **A2** has X⁵, X⁶, X⁷, X⁸, and X⁹ as D Fluorobenzene **A2** is reacted with CO₂ and an organolithium reagent to provide 2-fluorobenzoic acid **A3**, which is then combined with oxalyl chloride to provide 2-fluorobenzoyl chloride **A4**. To a suspension of hydroxyamidine **A5** is slowly added 2-fluorobenzoyl chloride **A4** to provide compound **A6**. This reaction is usually carried out with a base reagent, such as triethyl amine or diisopropylethylamine, in a solvent such as ethyl acetate. The hydroxyamidine **A5** has X¹ X², X³, and X⁴ independently as H. The acylated material **A6** is added to t-BuOH and heated to reflux where water is removed using a Dean-Stark trap over several hours. The ring-closure on the compound **A6**hydrolysis can be accomplished with a base reagent, such as sodium hydroxide, followed by treatment of the mixture with HCl to provide the hydrogen isotope-enriched 1,2,4-oxadiazole benzoic acid compound **A1**.

The hydrogen isotope-enriched 1,2,4-oxadiazole benzoic acid compound **A1** has X¹, X², X³, X⁴ as H, wherein X⁵, X⁶, X⁷, and X⁸ is D.

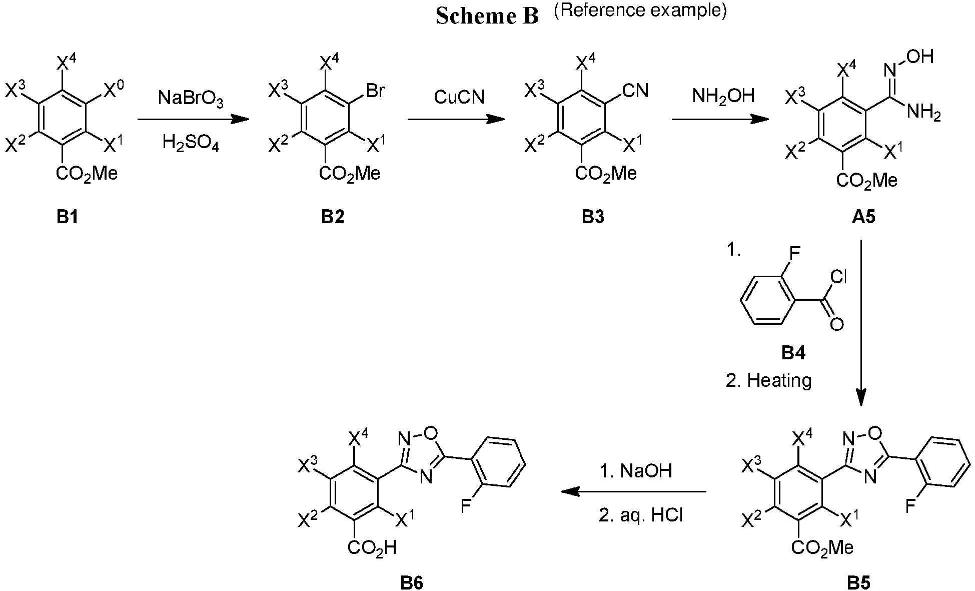

As depicted in Scheme B, the hydrogen isotope-enriched 3-(5-(2-fluorophenyl)-1,2,4-oxadiazol-3-yl)benzoic acid **B7** has X¹ X², X³, and X⁴ independently as H, D, or T, wherein at least one of X¹, X², X³,and X⁴ is D or T.

The methyl benzoate **B1** has X⁰, X¹ X², X³, and X⁴ independently as H, D, or T. A mixture of methyl benzoate **B1**, finely ground NaBrO₃ (0.95 eq.), and potassium sulfate in water is heated. To the mixture is added sulfuric acid over one hour and resulting solution is stirred for a further 18 hours. The mixture is cooled to ambient temperature and methyl 3-bromobenzoate **B2** is filtered and washed with cold water, and dried under vacuum.

To a solution of methyl 3-bromobenzoate **B2** in THF is added copper cyanide over one hour, and then stirred at elevated temperature. The mixture is cooled to ambient temperature and concentrated under reduced pressure. The crude product is purified using column chromatography to provide methyl 3-cyanobenzoate **B3**.

A solution of methyl 3-cyanobenzoate **B3** in *tert*-butanol is heated to 40 °C and then aqueous hydroxyl amine (1.05 eq.) is added over 4 hours. The mixture is stirred for a further 18 hours. The solution containing crude (Z)-methyl 3-(N'-hydroxycarbamimidoyl)benzoate **B4** is carried on to the next reaction step without further purification.

To the solution containing crude (Z)-methyl 3-(N'-hydroxycarbamimidoyl)benzoate **A5** cooled to 5 °C is added triethylamine (1.1 eq) and then 2-Fluorobenzoyl chloride **B4** is added over two hours while maintaining the temperature of the mixture at below 5 °C. The suspension is then heated to 80 °C and stirred for a further 18 hours. To the mixture is added H₂O and then cooled to ambient temperature. The mixture is filtered and the resulting solid is washed with cold water and dried under vacuum to provide methyl 3-(5-(2-fluorophenyl)-1,2,4-oxadiazol-3-yl)benzoate**B5**.

To a solution of methyl 3-(5-(2-fluorophenyl)-1,2,4-oxadiazol-3-yl)benzoate **B5** in *tert*-butanol is added aqueous sodium hydroxide solution (1.01 eq.). The mixture is stirred at elevated temperature. The solution is cooled to ambient temperature and then acidified with aqueous IN hydrochloric acid to reach a target pH of 2.0. The mixture is filtered and the resulting solid is washed with cold water and dried under vacuum to provide 3-(5-(2-fluorophenyl)-1,2,4-oxadiazol-3-yl)benzoic acid **B6**.

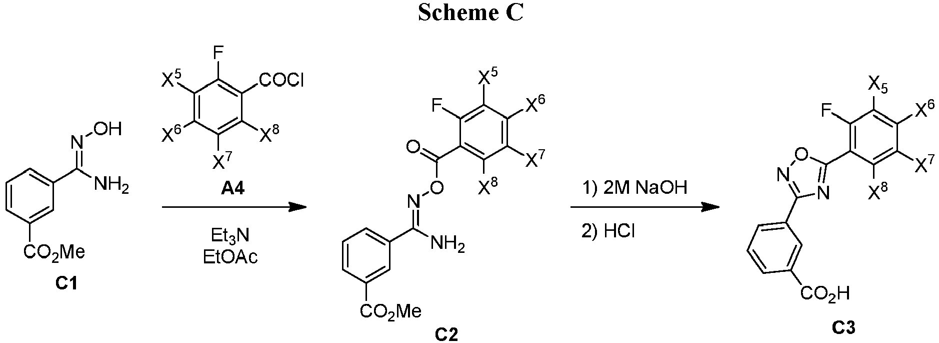

As depicted in Scheme C, the hydrogen isotope-enriched 3-(5-(2-fluorophenyl)-1,2,4-oxadiazol-3-yl)benzoic acid **A4** has X⁵, X⁶, X⁷, and X⁸ as D.

To a suspension of hydroxyamidine **C1** in ethyl acetate is added triethylamine at ambient temperature. The suspension is stirred for 10 minutes at ambient temperature followed by slow addition of 2-fluorobenzoyl chloride **A4** over 10 minutes at ambient temperature. The reaction mixture is stirred for 18 hours at ambient temperature. The resulting mixture is diluted with ethyl acetate (10 mL), followed by H₂O (2 mL x 2), and saturated sodium chloride. The organic layer is removed, dried with magnesium sulfate, then concentrated *in vacuo.* The crude reaction product is diluted with ethyl acetate and triturated in hexanes to obtain a crystalline product. The product is filtered, washed with 50% ethyl acetate and hexanes and dried *in vacuo* overnight to provide (Z)-methyl-3-(N'-((2-fluorobenzoyl)oxy)carbamimidoyl)benzoate **C2**.

To a solution (Z)-methyl-3-(N'-((2-fluorobenzoyl)oxy)carbamimidoyl)benzoate C2 in THF is added aqueous 2M NaOH. The reaction mixture is heated under reflux for 2 hours at 90 °C. The reaction mixture is diluted with *tert*-BuOH (2 mL) and then acidified by addition of aqueous 2M HCl until about pH 3.2 is obtained resulting in a white suspension. The suspension is stirred for 0.5 hour at ambient temperature and then filtered. The crude product is washed with hot H₂O (70 °C, 5 mL), followed by a mixture of t-BuOH/water. The crude product is dried under pressure and required no further purification, providing 3-(5-(2-fluorophenyl)-1,2,4-oxadiazol-3-yl)benzoic acid **C3**.

### 5. EXAMPLES

The following examples employ methodology which can be used to prepare all of the compounds of formula **I** or **II**, provided the appropriate reagents and substrates are utilized, and minor variations of the described conditions are maintained. Such variations would be easily performed by one of skill in the art without undue experimentation given the description below. In the following examples, compounds of formula **II** provided herein should be considered examples of the invention.

### 5.1.1 Example 1: Preparation of a Deuterium Isotope Enhanced Form of 3-(5-(2-fluorophenyl)-1,2,4-oxadiazol-3-yl)benzoic acid (1)

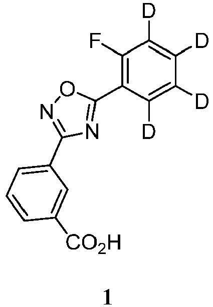

A deuterium isotope enhanced form of 3-(5-(2-fluorophenyl)-1,2,4-oxadiazol-3-yl)benzoic acid **(1)** was prepared by the reactions depicted in Scheme C, *infra.*

To a solution of fluorobenzene-D₅ **(2)** in THF was added 1.4M n-butyllithium in cyclohexanes at -72 °C over a period of 10 minutes. The resulting mixture was stirred for a further 2 hours at -72 °C. The reaction mixture was then diluted with ether (40 mL) at -50 °C followed by bubbling with bone-dry CO₂ at -72 °C for 1 hour. The reaction mixture was quenched with aqueous 3M HCl (20 mL) at -20 °C and then warmed to ambient temperature. The crude reaction mixture was diluted with ether (10 mL) and washed with H₂O. The organic layer was extracted twice with aqueous 1.5 M NaOH (50 mL × 2). The basic aqueous layer was twice washed with ether (100 mL × 2) and then acidified with aqueous 3M HCl until the pH was neutralized. The aqueous layer was twice extracted with ether (100 mL ×2), and the organic layers were combined and washed twice with H₂O (100 mL × 2), once with saturated sodium chloride (100 mL), dried with magnesium sulfate, filtered, and the organic solvent was concentrated *in vacuo* to provide 2-fluorobenzoic acid-D₄ **(3)** in 20% yield. The product was analyzed by LCMS.

To a solution of 2-fluorobenzoic acid-D₄ **(3)** in dichloromethane was added DMF (25 mL). The resulting mixture was stirred for 5 minutes at ambient temperature. To this mixture was added oxalyl chloride and the resulting mixture was stirred for 18 hours at ambient temperature. The solvent of the resulting mixture was evaporated *in vacuo* and the crude residue of 2-fluorobenzoyl chloride-D₄ **(4)** was carried on to the next reaction step without further purification.

To a suspension of hydroxyamidine **(5)** in ethyl acetate was added triethylamine at ambient temperature. The suspension was stirred for 10 minutes at ambient temperature followed by slow addition of 2-fluorobenzoyl chloride-D₄ **(4)** over 10 minutes at ambient temperature. The reaction mixture was stirred for 18 hours at ambient temperature. The resulting mixture was diluted with ethyl acetate (10 mL), followed by H₂O (2 mL x 2), and saturated sodium chloride. The organic layer was removed, dried with magnesium sulfate, then concentrated *in vacuo.* The crude reaction product was diluted with ethyl acetate and triturated in hexanes to obtain a crystalline product. The product was filtered, washed with 50% ethyl acetate and hexanes and dried *in vacuo* overnight to provide (Z)-methyl-3-(N'-((2-fluorobenzoyl)oxy)carbamimidoyl)benzoate-D₄ **(6)** in 93% yield. The product was analyzed by LCMS.

To a solution (Z)-methyl-3-(N'-((2-fluorobenzoyl)oxy)carbamimidoyl)benzoate-D₄ (6) in THF was added aqueous 2M NaOH. The reaction mixture was heated under reflux for 2 hours at 90 °C. The reaction mixture was diluted with *tert*-BuOH (2 mL) and then acidified by addition of aqueous 2M HCl until about pH 3.2 was obtained resulting in a white suspension. The suspension was stirred for 0.5 hour at ambient temperature and then filtered. The crude product was washed with hot H₂O (70 °C, 5 mL), followed by a mixture of t-BuOH/water. The crude product was dried under pressure and required no further purification, providing 3-(5-(2-fluorophenyl)-1,2,4-oxadiazol-3-yl)benzoic acid-D₄ **(1)** in 95% yield. The product was analyzed by NMR and LCMS.

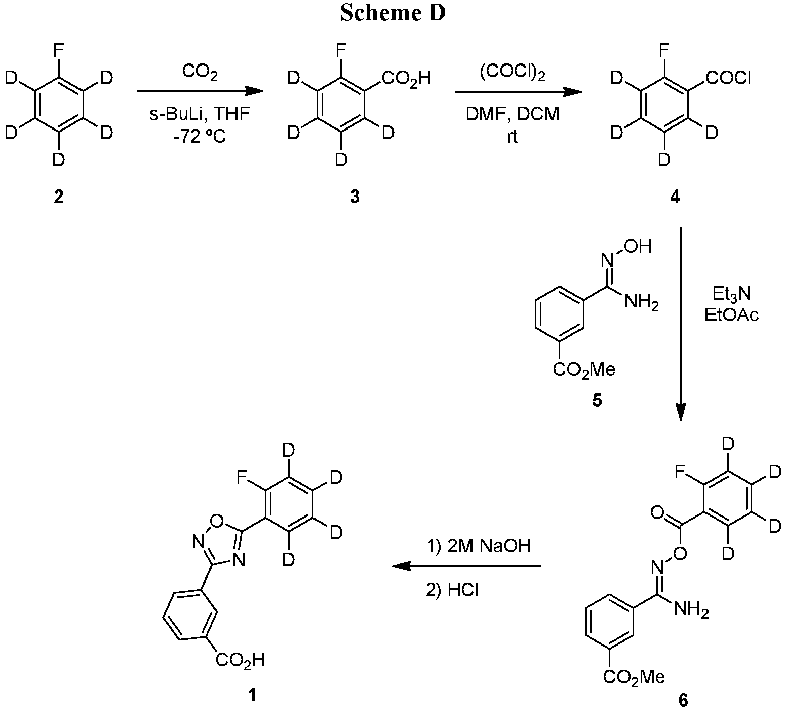

### 5.1.2 Example 3: Identification and characterization of compounds that promote nonsense suppression and/or modulate translation termination

The assays described above in Section 4.2 can be used in a high throughput screening methodology for identifying compounds that promote nonsense suppression and/or modulate translation termination. Compounds of formula **I** or **II** can be screened in the cell-based and biochemical assays. Compounds of formula **I** or **II** can be tested, resynthesized and tested again to confirm chemical structures. Compounds of formula **I** or **II** can be characterized further with the *in vitro* luciferase nonsense suppression assay. To ensure that the observed nonsense suppression activity of the selected compounds is not limited to the rabbit reticulocyte assay system, HeLa cell extract can be prepared and optimized (Lie & Macdonald, 1999, Development 126(22):4989-4996 and Lie & Macdonald, 2000, Biochem. Biophys. Res. Commun. 270(2):473-481).

### 5.1.3 Example 4: Characterization of compounds that increase nonsense suppression and produce functional protein

Compounds of formula **I** or **II** can be demonstrated to increase the level of nonsense suppression in the biochemical assay over untreated extracts. To determine whether compounds also function *in vivo,* a stable cell line harboring the UGA, UAA, or UAG nonsense-containing luciferase gene can be treated with selected compounds. Cells can be grown in standard medium supplemented with 1% penicillin-streptomycin (P/S) and 10% fetal bovine serum (FBS) to 70% confluency and split 1:1 the day before treatment. On the following day, cells can be trypsinized and 40,000 cells can be added to each well of a 96-well tissue culture dish. Serial dilutions of each compound can be prepared to generate a six-point dose response curve spanning 2 logs (30µM to 0.3 µM). The final concentration of the DMSO solvent can be measured. Cells treated with 1% DMSO can serve as the background standard, and cells treated with gentamicin can serve as a positive control.

### 5.1.4 Example 5: Readthrough of premature termination codons in cell-based disease models

To address the effects of the nonsense-suppressing compounds on mRNAs altered in specific inherited diseases, a bronchial epithelial cell line harboring a nonsense codon at amino acid 1282 (W1282X) can be treated with a compound of formula **I** or **II** (*e.g.,* 20µM) and CFTR function can be monitored as a cAMP-activated chloride channel using the SPQ assay (Yang et al., 1993, Hum Mol Genet. 2(8): 1253-1261 and Howard et al., 1996, Nat Med. 2(4):467-469). These experiments can show that cAMP treatment of these cells may result in an increase in SPQ fluorescence, consistent with stimulation of CFTR-mediated halide efflux. It would be expected that no increase in fluorescence would be observed when cells are not treated with compound or if the cells are not stimulated with cAMP. These results can indicate that the full-length CFTR expressed from this nonsense-containing allele following compound treatment also functions as a cAMP-stimulated anion channel, thus demonstrating that cystic fibrosis cell lines increase chloride channel activity when treated with a compound of formula **I** or **II**.

### 5.1.5 Example 6: Readthrough of premature termination codons in the mdx mouse

Similar to the procedure previously described (Barton-Davis et al., 1999, J Clin Invest. 104(4):375-381), a compound can be delivered by Alzet osmotic pumps implanted under the skin of anesthetized mice. Two doses of a compound of formula I or II can be administered. Gentamicin can serve as a positive control and pumps filled with solvent only can serve as the negative control. Pumps can be loaded with appropriate compound such that the calculated doses to which tissue was exposed are 10 µM and 20 µM. The gentamicin concentration can be calculated to achieve tissue exposure of approximately 200 µM. In an initial experiment, mice can be treated for 14 days, after which animals can be anesthetized with ketamine and exsanguinated. The tibialis anterior (TA) muscle of the experimental animals can then be excised, frozen, and used for immunofluorescence analysis of dystrophin incorporation into striated muscle. The presence of dystrophin in TA muscles can be detected by immunostaining, as described previously (Barton-Davis et al., 1999, J Clin Invest. 104(4):375-381).

### 5.1.6 Example 7: Sachet Formulation of 3-[5-(2-Fluoro-phenyl)-[1,2,4]oxadiazol-3-yl]-benzoic acid or a Pharmaceutically Acceptable Salt, Solvate or Hydrate Thereof

The mixture is packaged using a pouch or sachet that is comprised of multiple laminated layers that may include a paper layer, an aluminum foil layer and a surlyn layer. Each sachet can contain about 125 mg, about 250 mg, about 500 mg or about 1000 mg of a hydrogen isotope-enriched analogue of 3-[5-(2-fluoro-phenyl)-[1,2,4]oxadiazol-3-yl]-benzoic acid or a pharmaceutically acceptable salt, solvate or hydrate thereof. Excipients (and their proportions of the total formulation weight) optionally include either of the following as set forth in Table 4 and Table 5.

**Table 4. Formulation**

| **Ingredient** | **Weight %** |
|---|---|
| Hydrogen isotope-enriched analogue of 3-[5-(2-fluoro-phenyl)-[1,2,4]oxadiazol-3-yl]-benzoic acid or a pharmaceutically acceptable salt, solvate or hydrate thereof | 25.0 |
| Litesse^{®} Ultra | 24.75 |
| Polyethylene Glycol | 12.8 |
| Lutrol^{®} Micro | 3.7 |
| Mannitol | 25.0 |
| Hydroxyethyl Cellulose | 1.5 |
| Vanilla Flavor | 0.75 |
| Crospovidone | 5.0 |
| Cab-o-sil | 0.5 |
| Magnesium Stearate | 0.5 |
| Talc | 0.5 |

**Table 5. Formulation**

| **Ingredient** | **Weight %** |
|---|---|
| Hydrogen isotope-enriched analogue of 3-[5-(2-fluoro-phenyl)-[1,2,4]oxadiazol-3-yl]-benzoic acid or a pharmaceutically acceptable salt, solvate or hydrate thereof | 25.0 |
| Litesse^{®} Ultra | 25.65 |
| Polyethylene Glycol | 12.8 |
| Lutrol^{®} Micro | 3.7 |
| Mannitol | 25.0 |
| Hydroxyethyl Cellulose | 1.5 |
| Vanilla Flavor | 0.75 |
| Crospovidone | 5.0 |
| Cab-o-sil | 0.1 |
| Magnesium Stearate | 0.5 |

The sachet is then labeled to indicate the identity of the drug substance, the lot number, the amount of the drug substance, and the storage conditions (*e.g*., refrigeration at 2° to 8°C). Prior to administration, an appropriate amount of the drug product is reconstituted in an appropriate volume of a pharmaceutically acceptable solvent (*e.g.*, water, milk, a carbonated beverage, juice, apple sauce, baby food or baby formula). The drug product can be stored at room temperature for up to 48 hours prior to reconstitution.

### 5.1.7 Ophthalmic Formulations

Table 6 provides an ophthalmic formulation as a solution comprising a hydrogen isotope-enriched analogue of 3-[5-(2-fluoro-phenyl)-[1,2,4]oxadiazol-3-yl]-benzoic acid or a pharmaceutically acceptable salt, solvate or hydrate thereof in combination with tromethamine used as a cationic modifier.

**Table 6**

| **Ingredient** | **Concentration** |
|---|---|
| Hydrogen isotope-enriched analogue of 3-[5-(2-fluoro-phenyl)-[1,2,4]oxadiazol-3-yl]-benzoic acid or a pharmaceutically acceptable salt, solvate or hydrate thereof | 0.2 % |
| Tromethamine HCl | 1.0 % |
| Mannitol | 2.0 % |
| Boric Acid | 1.0 % |
| Disodium Edetate | 0.025 % |
| Benzalkonium Chloride | 0.01 % |
| NaOH/HCl (adjust pH) | pH 7.2 |
| Water (dilute to volume) | p.r.n. |

Table 7 provides an ophthalmic formulation as a solution comprising a hydrogen isotope-enriched analogue of 3-[5-(2-fluoro-phenyl)-[1,2,4]oxadiazol-3-yl]-benzoic acid or a pharmaceutically acceptable salt, solvate or hydrate thereof in combination with histidine used as a cationic modifier.

**Table 7**

| **Ingredient** | **Concentration** |
|---|---|
| Hydrogen isotope-enriched analogue of 3-[5-(2-fluoro-phenyl)-[1,2,4]oxadiazol-3-yl]-benzoic acid or a pharmaceutically acceptable salt, solvate or hydrate thereof | 0.1 % |
| Histidine HCl | 0.5 % |
| Sorbitol | 3.0% |
| Disodium Edetate | 0.025 % |
| Benzalkonium Chloride | 0.01 % |
| NaOH/HCl (adjust pH) | pH 6.5 |
| Water (dilute to volume) | p.r.n. |

Table 8 provides an ophthalmic formulation as a solution comprising a hydrogen isotope-enriched analogue of 3-[5-(2-fluoro-phenyl)-[1,2,4]oxadiazol-3-yl]-benzoic acid or a pharmaceutically acceptable salt, solvate or hydrate thereof in combination with Lysine used as a cationic modifier.

**Table 8**

| **Ingredient** | **Concentration** |
|---|---|
| Hydrogen isotope-enriched analogue of 3-[5-(2-fluoro-phenyl)-[1,2,4]oxadiazol-3-yl]-benzoic acid or a pharmaceutically acceptable salt, solvate or hydrate thereof | 0.05 % |
| Lysine HCl | 0.5 % |
| Mannitol | 4.0 % |
| Disodium Edetate | 0.025 % |
| Benzalkonium Chloride | 0.01 % |
| NaOH/HCl (adjust pH) | pH 7.5 |
| Water (dilute to volume) | p.r.n. |

Table 9 provides an ophthalmic formulation as a solution comprising a hydrogen isotope-enriched analogue of 3-[5-(2-fluoro-phenyl)-[1,2,4]oxadiazol-3-yl]-benzoic acid or a pharmaceutically acceptable salt, solvate or hydrate thereof in combination with DEAE-Dextran used as a cationic modifier.

**Table 9**

| **Ingredient** | **Concentration** |
|---|---|
| Hydrogen isotope-enriched analogue of 3-[5-(2-fluoro-phenyl)-[1,2,4]oxadiazol-3-yl]-benzoic acid or a pharmaceutically acceptable salt, solvate or hydrate thereof | 0.5 % |
| DEAE-Dextran | 0.5 % |
| Trehalose | 2.0 % |
| Boric Acid | 1.0 % |
| Disodium Edetate | 0.025 % |
| Benzalkonium Chloride | 0.01 % |
| NaOH/HCl (adjust pH) | pH 7.2 |
| Water (dilute to volume) | p.r.n. |

Table 10 provides an ophthalmic formulation as a solution comprising a hydrogen isotope-enriched analogue of 3-[5-(2-fluoro-phenyl)-[1,2,4]oxadiazol-3-yl]-benzoic acid or a pharmaceutically acceptable salt, solvate or hydrate thereof in combination with hydroxypropyl β -cyclodextrin used as a cationic modifier.

**Table 10**

| **Ingredient** | **Concentration** |
|---|---|
| Hydroxypropyl β-Cyclodextrin | 10% |
| Hydrogen isotope-enriched analogue of 3-[5-(2-fluoro-phenyl)-[1,2,4]oxadiazol-3-yl]-benzoic acid or a pharmaceutically acceptable salt, solvate or hydrate thereof | 0.5 % |
| Tromethamine HCl | 0.5 % |
| Mannitol | 2.0 % |
| Dextran | 1.0 % |
| Boric Acid | 1.0 % |
| Disodium Edetate | 0.025 % |
| Benzalkonium Chloride | 0.01 % |
| NaOH/HCl (adjust pH) | pH 7.2 |
| Water (dilute to volume) | p.r.n. |

## Claims

1. A compound, wherein the compound has the structure: 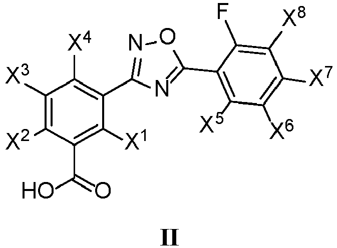
or a pharmaceutically acceptable salt, hydrate, solvate, clathrate, polymorph, or stereoisomer thereof,
wherein each of X¹, X², X³ and X⁴ is H; and
wherein each of X⁵, X⁶, X⁷ and X⁸ is D.

2. A pharmaceutical composition comprising a compound of claim 1 and a pharmaceutically acceptable carrier, excipient or diluent.

3. A compound of claim 1 or a pharmaceutical composition of claim 2 for use in a method for treating a disease or disorder associated with a premature stop codon in a patient, wherein the disease or disorder associated with a premature stop codon is a cancer, a muscular dystrophy, a lysosomal storage disease, an autoimmune disease, a blood disease, a collagen disease, diabetes, a neurodegenerative disease, a proliferative disease, a cardiovascular disease, a pulmonary disease, an inflammatory disease, a central nervous system disease, an ocular disease, a metabolic disorder, a skin disease, a neuropathic disease, or a neuromuscular disorder.

4. The compound or the pharmaceutical composition for use of claim 3, wherein the disease or disorder associated with a premature stop codon is muscular dystrophy; and wherein the muscular dystrophy is Duchenne muscular dystrophy.

## Patentansprüche

1. Verbindung, wobei die Verbindung die folgende Struktur aufweist: 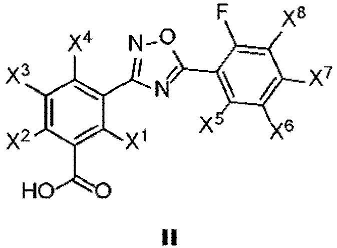
oder ein pharmazeutisch verträgliches Salz, Hydrat, Solvat, Clathrat, Polymorph oder Stereoisomer davon,
wobei jedes von X¹, X², X³ und X⁴ H ist; und
wobei jedes von X⁵, X⁶, X⁷ und X⁸ D ist.

2. Pharmazeutische Zusammensetzung, umfassend eine Verbindung nach Anspruch 1 und einen pharmazeutisch verträglichen Träger, Hilfsstoff oder Verdünner.

3. Verbindung nach Anspruch 1 oder eine pharmazeutische Zusammensetzung nach Anspruch 2 zur Verwendung in einem Verfahren zur Behandlung einer Erkrankung oder Störung in Verbindung mit einem vorzeitigen Stopcodon bei einem Patienten, wobei die Erkrankung oder Störung in Verbindung mit einem vorzeitigen Stopcodon ein Krebs, eine Muskeldystrophie, eine lysosomale Speicherkrankheit, eine Autoimmunkrankheit, eine Blutkrankheit, eine Kollagenkrankheit, Diabetes, eine neurodegenerative Erkrankung, eine proliferative Erkrankung, eine Herz-Kreislauf-Erkrankung, eine Lungenkrankheit, eine Entzündungserkrankung, eine Erkrankung des zentralen Nervensystems, eine Augenerkrankung, eine Stoffwechselstörung, eine Hautkrankheit, eine neuropathische Erkrankung, oder eine neuromuskuläre Störung ist.

4. Verbindung oder pharmazeutische Zusammensetzung zur Verwendung nach Anspruch 3, wobei die Erkrankung oder Störung in Verbindung mit einem vorzeitigen Stopcodon Muskeldystrophie ist; und wobei die Muskeldystrophie Duchenne-Muskeldystrophie ist.

## Revendications

1. Composé, dans lequel le composé présente la structure : 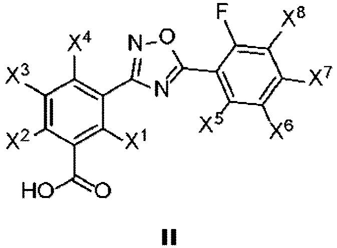
ou un sel, un hydrate, un solvate, un clathrate, un polymorphe, ou un stéréoisomère pharmaceutiquement acceptable de celui-ci,
dans lequel chacun de X¹, X², X³ et X⁴ est H ; et
dans lequel chacun de X⁵, X⁶, X⁷ et X⁸ est D.

2. Composition pharmaceutique comprenant un composé selon la revendication 1 et un vecteur, un excipient ou un diluant pharmaceutiquement acceptable.

3. Composé selon la revendication 1 ou composition pharmaceutique selon la revendication 2 pour utilisation dans un procédé de traitement d'une maladie ou d'un trouble associé à un codon stop prématuré chez un patient, dans lesquels la maladie ou le trouble associés à un codon stop prématuré est un cancer, une dystrophie musculaire, une maladie de surcharge lysosomale, une maladie auto-immune, une maladie du sang, une maladie du collagène, un diabète, une maladie neurodégénérative, une maladie proliférative, une maladie cardiovasculaire, une maladie pulmonaire, une maladie inflammatoire, une maladie du système nerveux central, une maladie oculaire, un trouble métabolique, une maladie cutanée, une maladie neuropathique, ou un trouble neuromusculaire.

4. Composé ou composition pharmaceutique pour utilisation selon la revendication 3, dans lesquels la maladie ou le trouble associés à un codon stop prématuré est dystrophie musculaire ; et dans lequel la dystrophie musculaire est la myopathie de Duchenne.
